# EUROPEAN PATENT SPECIFICATION

(11) **EP 2 519 250 B1**
(45) Date of publication and mention of the grant of the patent: **01.06.2016**
(21) Application number: 10807562.3
(22) Date of filing: 30.12.2010
(51) Int. Cl.: A61K 38/12, A61K 38/21, C07K 5/00, C07K 14/57, A61P 37/00, A61P 35/00, A61P 31/12, A61P 43/00, A61P 1/16, A61P 9/10, A61P 11/00

(54) **Interferon analogs**
Interferon-Analoga
Analogues d'interféron

(30) Priority: 10.02.2010 US 302973 P; 31.12.2009 EP 09181049
(43) Date of publication of application: 07.11.2012
(73) Proprietor: Biorion Technologies B.V., 9713 GX Groningen (NL)
(72) Inventor: POELSTRA, Klaas, NL-9713 AV Groningen (NL); PRAKASH, Jai, NL-9713 AV Groningen (NL); BELJAARS, Eleonora, NL-9713 AV Groningen (NL); BANSAL, Ruchi, NL-9713 AV Groningen (NL)
(74) Representative: V.O.
(86) International application number: PCT/NL2010/050897
(87) International publication number: WO 2011/081523

(56) References cited:
- WO-A1-00/23113
- WO-A2-2004/005341
- WO-A2-2008/068621
- US-A- 5 770 191
- WERNER I HAGENS ET AL: "Targeting 15d-Prostaglandin J2 to Hepatic Stellate Cells: Two Options Evaluated", PHARMACEUTICAL RESEARCH, KLUWER ACADEMIC PUBLISHERS-PLENUM PUBLISHERS, NE LNKD- DOI:10.1007/S11095-006-9175-2, vol. 24, no. 3, 24 January 2007 (2007-01-24), pages 566-574, XP019483489, ISSN: 1573-904X
- AHMED CHULBUL M I ET AL: "Peptide mimotics of gamma interferon possess antiviral properties against vaccinia virus and other viruses in the presence of poxvirus B8R protein", JOURNAL OF VIROLOGY, vol. 79, no. 9, May 2005 (2005-05), pages 5632-5639, XP002586641, ISSN: 0022-538X
- AHMED CHULBUL M ET AL: "IFN mimetic as a therapeutic for lethal vaccinia virus infection: Possible effects on innate and adaptive immune responses", JOURNAL OF IMMUNOLOGY, vol. 178, no. 7, April 2007 (2007-04), pages 4576-4583, XP002586642, ISSN: 0022-1767
- BELJAARS LEONIE ET AL: "The preferential homing of a platelet derived growth factor receptor-recognizing macromolecule to fibroblast-like cells in fibrotic tissue.", BIOCHEMICAL PHARMACOLOGY, vol. 66, no. 7, 1 October 2003 (2003-10-01), pages 1307-1317, XP002586643, ISSN: 0006-2952
- SUBRAMANIAM PREM S ET AL: "The COOH-terminal nuclear localization sequence of interferon gamma regulates STAT1alpha nuclear translocation at an intracellular site", JOURNAL OF CELL SCIENCE, vol. 113, no. 15, August 2000 (2000-08), pages 2771-2781, XP002586644, ISSN: 0021-9533
- TEMMING ET AL: "RGD-based strategies for selective delivery of therapeutics and imaging agents to the tumour vasculature", DRUG RESISTANCE UPDATES, CHURCHILL LIVINGSTONE, EDINBURGH, GB LNKD- DOI:10.1016/J.DRUP.2005.10.002, vol. 8, no. 6, 1 December 2005 (2005-12-01), pages 381-402, XP005328751, ISSN: 1368-7646

## Description

The invention relates to the field of medicine. Among others, it relates to biologically active analogs of interferons (IFNs) which show less unwanted side-effects and to the therapeutic uses thereof.

About ten distinct IFNs have been identified in mammals; seven of these have been described for humans. They are typically divided among three IFN classes: Type I IFN, Type II IFN, and Type III IFN. All type I IFNs bind to a specific cell surface receptor complex known as the IFN-α receptor (IFNAR) that consists of IFNAR1 and IFNAR2 chains. The type I interferons present in humans are IFN-α, IFN-β and IFN-ω. Interferon type II binds to IFNGR. In humans this is IFN-γ. By interacting with their specific cell surface receptors, IFNs activate signal transducer and activator of transcription (STAT) complexes; STATs are a family of transcription factors that regulate the expression of certain immune system genes. Some STATs are activated by both type I and type II IFNs. However each IFN type can also activate unique STATs (Platanias, L. C. 2005 Nature reviews. Immunology 5 (5): 375-386).

IFNs belonging to all IFN classes are very important for fighting viral infections. Although they are named after their ability to "interfere" with viral replication within host cells, IFNs have other functions: they activate immune cells, such as natural killer cells and macrophages; they increase recognition of infection or tumor cells by up-regulating antigen presentation to T lymphocytes; and they increase the ability of uninfected host cells to resist new infection by virus. Certain host symptoms, such as aching muscles and fever, are related to the production of IFNs during infection.

IFNγ is a pleiotropic cytokine produced by the activated immune cells. It acts through the IFNγ receptor that is expressed nearly on all cell types, however it displays a strict species specificity. IFNγ has been applied for the treatment of immunological, viral and cancer diseases (Younes and Amsden, J Pharm Sci 2002) with significant effects. In addition, several studies have demonstrated the potential role of IFNγ in renal and liver fibrosis (Kidney Int. 1999 ;56:2116-27, Hepatology 1996 23:1189-99). Unfortunately, the short circulation half-life and undesirable systemic side effects of currently available interferons have limited its clinical application and even halted clinical trials. Many attempts have been made to circumvent these problems e.g. by incorporating IFNγ into liposomes, microspheres and elastomers (Pharm Res. 2000 17 :42-8, Pharm Res. 1996 13:1464-75, J Control Release. 2005 102:607-17). Cell-specific delivery approaches have not been used so far. This is not surprising in view of the fact that such an approach is generally deemed impossible for cytokines which need to be delivered to their own receptors to elicit a biological effect, so that they always end up in target cells that express these receptors. Delivery to other target receptors will therefore in most cases be useless and at best lead to uptake in other cell types causing loss of activity or further diversification of adverse effects.
However, the present inventors recognized that the structure of INF offers unique targeting opportunities since the molecule contains a receptor binding moiety which is species-specific and a signalling moiety which is non-species specific and which acts intracellularly. It was surprisingly found that that the unique structure of INFγ allows delivery of the signalling moiety of IFNγ to another target receptor, provided that this new target receptor allows intracellular release of this signalling moiety and subsequent activation of the intracellular/nuclear INFγ signalling pathway. For example, a truncated IFNγ mimetic targeted to the Platelet Derived Growth Factor (PDGF) receptor displayed significantly less systemic side effects in an acute liver injury mouse model when compared to full length IFNγ or non-targeted IFNγ mimetic.

Accordingly, the invention relates to an analog of interferon (IFN), wherein the moiety mediating binding to its natural receptor is at least functionally disrupted and wherein the analog comprises a signaling moiety capable of mediating intracellular IFN activity, said signaling moiety being provided at its N-terminus, optionally via a linker, with at least one targeting domain capable of binding to a cell surface receptor other than the IFN receptor. Preferably, the analog is an IFN gamma (IFNγ) analog.

Interferon analogs provided with a heterologous targeting moiety are known in the art. WO2008/068621 discloses a conjugation product of IFNγ and a targeting moiety, such as a tumor targeting moiety or a tumor vasculature targeting moiety. The prior art only discloses conjugation to full length IFNγ, which will still bind to its native receptor causing adverse effects.

EP0844252 aims to provide cyclic peptides and their preparation process, which allow subsequent chemical grafting on coupling on said cyclic peptides, i.e. their attachment to a solid support, to a high molecular weight compound, to a marker and/or to other cyclic peptides, in order to provide new or improved biotechnological applications, particularly in the field of affinity chromatography, immunisation, development of diagnostic tests, vaccines and pharmaceutical compositions. It teaches the coupling of cyclic peptide ligands to biological molecules, among others to interferons. No mention is made in EP0844252 about the use of a truncated IFN molecule lacking a functional binding domain to its natural receptor.

An interferon analog of the present invention has clear and unexpected advantages over the use of a (modified) full length interferon, not only with respect to its therapeutic use (less side-effects, increased efficacy, reduced antigenicity because of lower molecular weight) but also to its production process (recombinant synthesis).

The functional disruption of the moiety mediating binding to its natural receptor can be achieved by deleting, inserting, substituting one or more relevant amino acid residues in the receptor binding domain. Interferon receptor binding domains have been identified and are known in the art. For example, binding of murine IFNγ to the INFγ receptor can be abolished by at least partially deleting the first 40, 30 or 20 N-terminal residues.

In one embodiment, the analog is a truncated IFNγ polypeptide which only contains the residues involved in intracellular signalling. As used herein, intracellular signalling may comprise nuclear translocation and/or anti-viral activity.

Preferably, the signaling moiety mediating intracellular activity comprises a polybasic nuclear localization signal (NLS) motif as found in the C-terminus of human and murine IFN (Subramaniam et al. 2000, J. Cell Science 113, 2771-2781). This NLS motif is thought to form a complex with a signal transducer and activator of transcription (STAT); STATs are a family of transcription factors that regulate the expression of certain immune system genes. Some STATs are activated by both type I and type II IFNs. However each IFN type can also activate unique STATs (Platanias, L. C. 2005 Nature reviews. Immunology 5 (5): 375-386) STAT activation initiates the most well defined cell signaling pathway for all IFNs, the classical Janus kinase-STAT (JAK-STAT) signaling pathway.In this pathway, JAKs associate with IFN receptors and, following receptor engagement with IFN, phosphorylate both STAT1 and STAT2. As a result, an IFN-stimulated gene factor 3 (ISGF3) complex forms - this contains STAT1, STAT2 and a third transcription factor called IRF9 - and moves into the cell nucleus. Inside the nucleus, the ISGF3 complex binds to specific nucleotide sequences called IFN-stimulated response elements (ISREs) in the promoters of certain genes; this induces transcription of those genes.

An analog provided herein may comprise the polybasic NLS motif comprises the amino acid sequence (R)KRXRS(R), wherein X is any amino acid residue, preferably wherein X is R, K, S or T. Preferably, the NLS motif is present at the C-terminal end of the analog. In one embodiment, it comprises the sequence, preferably the C-terminal sequence RKRKRSR, KSKRSR, KRTRS or KRTRSQ. In a specific aspect, the signaling moiety comprises or consists of a sequence selected from the group consisting of
(a) the amino acid sequence KFEVNNPQVQRQAFNELIRVVHQLLPESSLRKRKRSR;
(b) the amino acid sequence YSVTDLNVQRKAIHELIQVMAELSPAAKTGKRTRSQ or YSVTDLNVQRKAIHELIQVMAELSPAAKTGKRKRSQ;
(c) the amino acid sequence AKFEVNNPQIQHKAVNELIRVIHQLSPESSLRKRKRSRC
(d) a stretch of at least 10, preferably at least 15, contiguous amino acids of the sequence of (a), (b) or (c);
(e) an amino acid sequence showing at least 70%, preferably at least 80%, more preferably at least 90% identity to a) or b) or c) provided that the intracellular signaling activity is maintained; and
(f) the amino acid sequence under (a) or (b) or (c) wherein at most 10, preferably at most 8, more preferably at most 5 amino acid residues are deleted, added or substituted, provided that the signaling activity, for example nuclear translocation, is maintained.
(g) the consensus sequence VxxxxVQRxAxxELIxVxxxLxPxxxxxKRxRS wherein x is any amino acid residue;
(h) the consensus sequence VxxxxxQxxAxxELIxVxxxLxPxxxxxKRKRS wherein x is any amino acid residue;
(i) the consensus sequence Vxxxx[V/I]Q[R/H] [Q/K]A[F/V/I] [N/H] ELI[R/Q]Vx[H/A] [Q/E]L[L/S]P[E/A] [S/A] [S/A] [L/ K]xxKRKRS wherein x is any amino acid residue;
The amino acid sequence under (a) represents a truncated murine INFγ sequence, the sequence under (b) is a human homologue and the sequence under (c) is the rat homolog. Preferably, an analog of the invention comprises a stretch of at least 10, preferably at least 15, more preferably at least 20 contiguous amino acids of the sequence of (a) or (b) or (c). In one embodiment, said stretch comprises the N-terminal sequence of the sequence under (a) or (b) or (c). In another embodiment, it comprises the C- terminal sequence of the sequence under (a) or (b) or (c), preferably at least the last 15 amino acids. In yet another embodiment, the stretch comprises an internal sequence of the sequence under (a) or (b) or (c). Exemplary sequences are LLPESSLRKRKRSR, KFEVNNPQVQRQ, QAFNELIRVVHQLL, MAELSPAAKTGKRTRSQ, YSVTDLNVQRKAI, KAIHELIQVMAELS.

The skilled person will understand that variants with one or more amino acid modifications to the sequences under (a) or (b) or (c) are also within the scope of the invention. the amino acid sequence under (a) or (b) or (c) wherein at most 10, preferably at most 8, more preferably at most 5 amino acid residues are deleted, added or substituted, provided that the signaling activity, for example nuclear translocation, is maintained.

Alignment of the human and murine INFγ sequences shows that 15 out of the 36 residues (41%) are identical and 24 out of the 36 residues (66%) are positively charged. The identity match is done according to clustal W alignment software.

| | |
|---|---|
| huInterferon gamma | 4 YSVTDLN VQR K AIHE L I Q V MAELS PAAKTG KRTRSQ 39 |
| | + V + VQR + A + E L I + V + + L P + KR RS+ |
| MuInterferon gamma | 3 FEVNNPQ VQRQ AFNE L IRV VH QLL PESS LRKRK RS R 38 |

The line in between the sequences indicates the conserved residues, yielding a consensus sequence recited under (g) herein above. Alignment of the human, rat and mouse interferon gamma can provide the consensus sequences under (h) and (i). In one embodiment, an analog comprises a signalling moiety according to the consensus sequence (g), (h) or (i) mentioned above. Other useful sequences include an amino acid sequences showing at least 70%, preferably at least 80%, more preferably at least 90%, most preferably at least 95% identity to (a) or (b) or (c) provided that the intracellular signaling activity is maintained.

In a specific aspect, the analog comprises a signalling moiety according to the consensus sequence:
Xaa¹Xaa²Xaa³Xaa⁴ Val Xaa⁵ Xaa⁶ Xaa⁷ Xaa⁸ Xaa⁹ Gln Xaa¹⁰ Xaa¹¹ Ala Xaa¹² Xaa¹³ Glu Leu Ile Xaa¹⁴ Val Xaa¹⁵ Xaa¹⁶ Xaa¹⁷ Leu Xaa¹⁸ Pro Xaa¹⁹ Xaa²⁰ Xaa²¹ Xaa²² Xaa²³Lys Arg Lys Arg Ser Xaa²⁴ Xaa²⁵, wherein
Xaa¹, Xaa², Xaa³ Xaa⁴ Xaa⁶ Xaa¹¹, Xaa¹³, Xaa¹⁴, Xaa¹⁶ Xaa¹⁷, Xaa¹⁸ Xaa¹⁹ Xaa²⁰ Xaa²¹ Xaa²² Xaa²³ Xaa²⁴ and Xaa²⁵ is any amino acid residue Xaa⁵ is a polar, uncharged residue such as Asn or Thr Xaa⁷ is a non-polar, hydrophobic residue such as Pro or Leu Xaa⁸ is a polar, uncharged residue such as Gln or Asn Xaa⁹ is a non-polar hydrophobic residue such as Val, Ile or Leu Xaa is a polar, basic residue such as Arg, His or Lys Xaa¹² is a non-polar hydrophobic residue such as Phe, Val or Ile Xaa¹⁵ is a non-polar hydrophobic residue such as Val, Ile, Met
Preferably, an analog comprises as signalling moiety a sequence corresponding to residues 95-133 in murine IFNγ or residues 95-134 in human IFNγ. This sequence has antiviral activity (Mujtaba et al. 2006, Clinical and Vaccine Immunology, Vol. 13, No.8, p.944-952).

The signaling activity of an analog can be readily determined by methods known in the art. For example, the capacity of the analog to induce nuclear translocation of STAT1α when taken up intracellularly by a murine macrophage cell line can be determined as described by Subramaniam et al. Signaling activity can also be assessed by complex formation with the transcription factor Statlalpha and the nuclear importer of Statlalpha, the importin-alpha analog NPI-1. See in particular Subramaniam et al. (2001, J. Interferon Cytokine Res. 21(11):951-959). Other suitable *in vitro* assays include NO-production in murine macrophages such as the RAW cell line. In one embodiment, the analog shows at least 30%, preferably at least 50%, more preferably at least 75% of the (*in vitro*) activity of its full length interferon counterpart. Cell specific targeting can significantly enhance the *in vivo* efficacy of an analog showing relatively low activity *in vitro.*

As mentioned herein above, an analog of the present invention is characterized by the absence of a functional IFN-receptor binding domain and the presence of a targeting domain capable of binding to an alternative receptor which can mediate intracellular uptake of the analog, for example a receptor which enters the endocytic pathway upon ligand binding and/or as part of constant receptor turnover. As will be understood, the alternative or "secondary" receptor to be targeted should have some degree of cell type specific expression. Ubiquitously expressed receptors are less suitable candidates. Furthermore, the receptor must be expressed on a cell which is responsive to the intracellular signaling moiety, e.g. in case of an IFNγ analog it must contain an IFNγ-responsive element.

Preferably, the targeting domain can bind to a receptor that is specific for fibroblast and fibroblast-like cells, such as for example myofibroblasts, portal fibroblasts, mesangial cells, interstitial fibroblasts, alveolar fibroblasts or stromal cells. Also envisaged are receptors expressed on tumor cells. An IFNγ - analog can induce apoptosis in tumor cells, which can be specifically targeted via a secondary receptor expressed on tumor cells. In one embodiment, the receptor is selected from the group consisting of the PDGF receptors, collagen type VI receptor, cytokine receptors including TGFβ receptor, TNFα receptor and the IL1β receptor, Insulin growth factor receptors, VEGF receptors and chemokine receptors (e.g. CXCR4). Very good results were obtained when the analog was targeted to the PDGF receptor, preferably the PDGF-β receptor. Other suitable targeting domains include cell adhesion peptides, such as the tripeptide Arg-Gly-Asp (RGD) which was originally identified as the sequence within fibronectin that mediates cell attachment. The RGD motif has now been found in numerous other proteins and supports cell adhesion in many, but not all, of these. The integrins, a family of cell-surface proteins, act as receptors for cell adhesion molecules. A subset of the integrins recognize the RGD motif within their ligands, the binding of which mediates both cell-substratum and cell-cell interactions. RGD-containing analogs may be used as therapeutic agents for the treatment of diseases such as thrombosis and cancer.

In a specific aspect, the targeting domain targets can bind to a receptor for an oligosaccharide or a glycoprotein, preferably the asialoglycoprotein (ASGP) receptor or the mannose receptor (CD 206). For example, the targeting domain is an oligosaccharide, preferably mannose or lactose, conjugated to a carrier molecule. In another embodiment, mannose-6-phosphate (M6P) or a derivative thereof can be used as targeting domain for the M6P/IGF II receptor. See for example EP1117443.

The targeting domain is a preferably a proteinaceous substance (peptide) such that the analog as a whole is a fusion polypeptide which can be readily produced e.g. recombinantly. However, non-proteinaceous other types of targeting domains are also envisaged such as saccharides, lipids, nucleic acids, synthetic molecules, and the like.

In one aspect, the targeting domain comprises at least one cyclic peptide portion. Peptides can be cyclised by various means, including cysteine-disulfide or lanthionine bridge formation. Cyclic peptides useful for targeting an analog of the invention to a desired cell type are described in the art. For example, EP1117443 discloses a cyclic peptide comprising at least one sequence encoding a cell receptor recognising peptide (RPR). In one embodiment, an analog of the invention comprises in its (cyclised or linear) targeting domain at least an amino acid sequence selected from the group consisting of RGD, KPT, SRN, NLI and LID.

A preferred embodiment relates to analogs having a targeting domain which comprises multiple receptor binding sequences, preferably in the form of at least one tandem repeat of a (cyclic) peptide portion. The tandem repeat may comprise two cyclic peptide portions, preferably identical cyclic peptide portions, connected via a linker of 1 to 5 amino acids. This is of particular advantage for targeting a receptor which is active as a dimer, such as the PDGF, TGFβ or IL-10 receptor.
In one embodiment, the targeting domain comprises one, preferably two, copie(s) of the amino acid sequence X₁SRNLIDX₂, wherein X₁ and X₂ denote moieties which together can form a (peptidic) bond such that a cyclic structure is formed wherein the sequence SRNLID is part of the ring. The two copies are preferably spaced by a linker sequence of 1 to 7 amino acids. Preferably, X₁ and X₂ are Cys residues. The inventors found that a targeting domain comprising the amino acid sequence CSRNLIDC-linker- CSRNLIDCS, wherein the linker is an amino acid sequence of 1 to 7, like 1, 2, 3,4, 5, 6 or 7, amino acid residues, is very effective in binding to the dimeric PDGFβ receptor. For example, they performed a comparative experiment wherein two cyclic peptides capable of binding to the PDGF-receptor were linked to each other via a spacer consisting of 6 amino acids whereas in another construct these two cyclic peptides were coupled via a spacer of 11 amino acids. In both constructs the spacer contained a lysine residue to allow coupling of a drug or tracer. Both bicyclic constructs were labeled with FITC and added to 3T3 cell cultures, expressing the PDGF-receptor and incubated for 2 hrs to examine the binding. Following incubation and staining with an antibody against FITC, immunohistochemical data showed significant binding of the construct comprising the spacer of 6 amino acids, whereas only low binding was observed with the construct containing the longer spacer of 11 amino acids.

In one embodiment, the linker may consist of 4 or 5 amino acid residues, preferably selected from the group of Gly, Ala, Ser and Thr residues, more preferably at least 3 of them being a glycine residue. Preferred linkers consist of the sequence K(GS)ₘGG wherein m is 1 or 2. As a specific example, the targeting domain comprises or consists of the amino acid sequence CSRNLIDCKGSGGCSRNLIDCS or CSRNLIDCKGSGSGGCSRNLIDCS. In another embodiment, the linker consists 5 or 6 amino acid residues, preferably selected from the group of Asp, Lys, Gly, Ala, Ser and Thr residues. Good results were obtained with a linker of 4 to 7 residues, at least 4, preferably at least 5, being a glycine residue. Other suitable linkers include a sequence of 4 to 7 residues, the residues being selected from Gly and Asp residues, e.g. [GₙDₘ] wherein n+m is from 4 to 7, wherein n ≥ 4 and M an integer between 0 and 3. In a specific embodiment, the targeting domain comprises or consists of the amino acid sequence CSRNLIDC[GₙDₘ] CSRNLIDC, wherein n+m is from 4 to 7, wherein n ≥ 4 and M an integer between 0 and 3. For example, the targeting domain consists of or comprises the sequence CSRNLIDCGGGDGGCSRNLIDC, CSRNLIDCGGDGGCSRNLIDC, CSRNLIDCGDDGGCSRNLIDC or CSRNLIDCGGGGGGCSRNLIDC.

The targeting domain may be attached to the signalling domain by any means, for instance by a linker or spacer sequence. Suitable linker sequences are typically up to 15, preferably up to 10 amino acid residues in length. Polyalanine linkers may be used. For instance, provided herein is an interferon gamma analog, consisting of the sequence CSRNLIDCKGSGGCSRNLIDCSAAAAKFEVNNPQVQRQAFNELIR VVHQLLPESSLRKRKRSR, CSRNLIDCKGSGSGGCSRNLIDCSAAA AKFEVNNPQVQRQAFNELIRVVHQLLPESSLRKRKRSR or CSRNLIDC GGGDGGCSRNLIDCSAAA KFEVNNPQVQRQAFNELIRVVHQLLPESSLRKRKRSR

The targeting domain may also be attached to the signalling domain via a carrier molecule, e.g. albumin. This is especially advantageous if the targeting domain is a non-proteinaceous substance e.g. an oligosaccharide such as mannose or lactose. In one embodiment, the carrier molecule comprises free reactive groups like hydroxyl, amine and/or sulphate. The size of the carrier is preferably an endogenous plasma protein, like albumin, lactoferrin or fibronectin.

The invention also relates to a conjugate comprising a compound of interest, e.g. a biologically active molecule, conjugated to a bicyclic PDGF-targeting domain, said targeting domain comprising two copies of the amino acid sequence X₁SRNLIDX₂, wherein X₁ and X₂ denote moieties which together can form a bond, like a peptidic or disulphide bond, such that a bicyclic structure is formed wherein the sequence SRNLID is part of each ring. Preferably, X₁ and X₂ are Cys residues allowing for cyclisation via disulphide bond formation. A conjugate according to the invention is characterized in that the two copies are spaced by a linker sequence of 2 to 7 amino acids. It was surprisingly found that this specific spacer length allows for highly efficient targeting of the PDGF receptor, which is active as a dimer. Thus, also provided is a conjugate comprising a compound of interest conjugated to a targeting domain, said targeting domain comprising the amino acid sequence X₁SRNLIDX₂-linker- X₃SRNLIDX₄, wherein the pair of X₁ and X₂ and the pair of X₃ and X₄ can form a (peptidic) bond such that a bicyclic structure is formed wherein the sequences SRNLID are part of a ring, and wherein the linker is an amino acid sequence consisting of 2 to 7 amino acid residues. As discussed herein above, the linker may consist of 4 or 5 amino acid residues. They can be selected from the group of Gly, Ala, Ser, Asp, Lys and Thr residues, preferably at least 3 of them being a glycine residue. Preferred linkers consist of the sequence K(GS)ₘGG wherein m is 1 or 2. As a specific example, the targeting domain comprises or consists of the amino acid sequence CSRNLIDCKGSGGCSRNLIDCS or CSRNLIDCKGSGSGGCSRNLIDCS. In another embodiment, the linker consists of 5 or 6 amino acid residues, preferably selected from the group of Asp, Gly, Ala, Ser and Thr residues. Good results were obtained with a linker of 4 to 7 residues, at least 4, preferably at least 5, being a glycine residue. Other suitable linkers include a sequence of 4 to 7 residues, the residues being selected from Gly and Asp residues, e.g. [GₙDₘ] wherein n+m is from 4 to 7, wherein n ≥ 4 and M an integer between 0 and 3. In a specific embodiment, the targeting domain comprises or consists of the amino acid sequence CSRNLIDC[GₙDₘ] CSRNLIDC, wherein n+m is from 4 to 7, wherein n ≥ 4 and M an integer between 0 and 3. For example, the targeting domain consists of or comprises the sequence CSRNLIDCGGGDGGCSRNLIDC, CSRNLIDCGGDGGCSRNLIDC, CSRNLIDCGDDGGCSRNLIDC or CSRNLIDCGGGGGGCSRNLIDC.

The use of the sequence SRNLID as cell targeting domain is known in the art. WO00/23113 discloses the conjugation of a cyclic peptide comprising a receptor recognizing peptide (RRP) to a carrier molecule being larger than 5000 Dalton, for instance serum albumin. An exemplary RRP is the PDGF receptor binding sequence XSRNLIDCX, wherein X denotes the location of cyclisation. It is taught that, within the cyclic peptide structure, multiple RRP sequences may be present. It is also disclosed to attach more than one (e.g. 5-15 cyclic peptides) to a carrier molecule. Hagens et al. (2007) Pharmaceutical Res. Vol. 24, pp. 566-574, show the delivery of cyclic peptide CSRNLIDC conjugated to albumin to hepatic stellate cells. Thus, the prior art conjugates all rely on attaching RRPs to a carrier molecule. The construction of conjugate of the invention, wherein the cyclic moieties are not attached to a carrier molecule but instead thereof placed in a tandem motif with a specific spacer length of 2 to 7 amino acids is not taught or suggested in the art. It was found that this well defined bicyclic structure enhances conjugate binding to the dimeric PDGF receptor as compared to a conjugate according to WO00/23113, wherein the number and spatial orientation of the multiple receptor binding sequences attached to the carrier are randomized and much less controlled. The tandem configuration fixing the distance between the two cyclic moieties in a conjugate of the invention is designed to interact optimally with the dimeric PDGF-receptor. Furthermore, the presence of the relatively large carrier molecule may decrease receptor interactions by steric hindrance

The bicyclic peptide can be prepared chemically or through recombinant techniques which provides production methods that are very favourable to the pharmaceutical industry. In addition, the bicyclic structure can be directly attached to a chemical entity (drug or tracer), polymer (eg Polyethylene glycol, PEG), small peptide or protein yielding a cell-specific low molecular weight compound that can easily penetrate into extravascular tissues. In particular for tumor targeting purposes, this tissue penetration may be very relevant.

Very good results were achieved with a linker consisting of 3 to 5 amino acid residues. In one embodiment, the conjugate comprises the amino acid sequence CSRNLIDC-linker- CSRNLIDCS (BiPPB) wherein the linker is an amino acid sequence of 2 to 7, preferably 3 to 5, amino acid residues. This bicyclic peptide is suitably used as low molecular weight targeting ligand with high affinity to the PDGF receptor (PDGF-R). The peptide can be prepared either by chemical or recombinant synthesis. The linker may comprise one or more amino acid residues with a reactive side chain that can be used for covalent attachment of a compound of interest, like a detectable label, a drug and/or diagnostic. Suitable reactive amino acids include lysine, serine and threonine, arginine, histidine, aspartic acid, glutamic acid, cysteine, asparagine, glutamine, tyrosine, methionine and tryptophan.

The biologically active moiety may have any type of useful biological activity, including cytokine, chemokine, or prostaglandin activity. It can be of proteinaceous or non-proteinaceous nature. For instance, the moiety is selected from the group selected from drugs, cytokines, chemokines, hormones, prostaglandins, and the like. Specific examples include PGE2, 15d-PGJ2, IL-10, IFNγ, truncated IFNγ. Proteinaceous moieties may conveniently be attached to the PDGF-R specific targeting domain by genetic fusion, at either the N- or C-terminus. In one embodiment, it is conjugated to the N-terminus.

An analog or conjugate according to the invention may be coupled to a core and/or carrier or delivery molecule by methods known in the art. Suitable cores or carriers include dendrimers, liposomes, and natural, synthetic or semi-synthetic polymers (branched or linear). Dendrimers have successfully proved themselves as useful additives in different routes of drug administration because they can render drugs greater water-solubility, bioavailability, and biocompatibility. See Chen et al., Journal of Pharmaceutical Sciences, Vol. 97 Issue 1, pg. 123 - 143. As an example, the invention provides an IFNγ-analog conjugated to a dendrimer or to a liposome. The liposome may containan (anti-cancer) drug.

In one embodiment, an interferon analog or PDGFR-targeted conjugate according to the invention is modified by conventional means to improve its pharmacological properties, like enhancing the efficacy and/or stability. In one embodiment, it is modified in order to enhance the half life by the attachment of at least one non antigenic polymer, for instance by a polymer selected from the group consisting of polyethylene glycols (PEGs) and derivatives thereof. PEGylation is routinely achieved by incubation of a reactive derivative of PEG with the target macromolecule. The covalent attachment of PEG to a drug or therapeutic protein can "mask" the agent from the host's immune system (reduced immunogenicity and antigenicity), increase the hydrodynamic size (size in solution) of the agent which prolongs its circulatory time by reducing renal clearance.

A further aspect of the invention relates to an isolated nucleic acid sequence encoding a proteinaceous interferon analog according to the invention or encoding a proteinaceous PDGFR-targeted conjugate as described herein above. The skilled person will be able to design and construct a suitable nucleic acid sequence e.g. a fusion construct that encodes both the targeting moiety and the biologically active moiety using standard recombinant DNA technology. The isolated nucleic acid sequence may be part of an expression vector, for example a vector designed for recombinant protein production in a bacterial or mammalian host cell. Also provided is a host cell comprising a nucleic acid sequence or a vector according to the invention, preferably wherein said host cell is a bacterial or mammalian host cell.
An analog or PDGF-targeted conjugate disclosed herein has improved properties with respect to its distribution within the body. More specifically, it allows directing a biologically active compound of interest to a cell of interest while maintaining the biological activity of that particular compound. To attain cell-specificity, these mediators are equipped with an address label that will increase their concentration around relevant target receptors in diseased tissue. A further embodiment therefore relates to a pharmaceutical composition comprising an IFN analog or a PDGF-targeted conjugate and a pharmaceutically acceptable carrier. A specific aspect relates to a pharmaceutical composition comprising a targeted IFNγ analog, preferably a PDGF-targeted IFNγ analog showing reduced side-effects as compared to non-targeted IFNγ. An exemplary pharmaceutical composition contains a peptide comprising or consisting of the sequence CSRNLIDCK(GS)ₘGGCSRNLIDCSAAAAKFEVNNPQ VQRQAFNELIRVVHQLLPESSLRKRKRSR wherein m is 1 or 2, for example CSRNLIDCKGSGSGGCSRNLIDCSAAAAKFEVNNPQVQRQAFNELIRVVHQLLPES SLRKRKRSR. Another exemplary pharmaceutical composition comprises a conjugate comprising a biologically active molecule conjugated to a targeting domain, said targeting domain comprising the amino acid sequence CSRNLIDC-linker-CSRNLIDCS, wherein the linker is an amino acid sequence of 2 to 7, preferably 3 to 5, amino acid residues.

Also provided is the use of an IFN (γ) analog according to the invention for the manufacture of a medicament for the therapeutic or prophylactic treatment of a disease selected from cancer, viral diseases, fibrotic disease, sclerotic disease and chronic or acute inflammatory diseases. Exemplary diseases include glomerulosclerosis, interstitial fibrosis, lung fibrosis (idiopathic pulmonary fibrosis), atherosclerosis, rheumatoid arthritis, Crohns disease, colitis ulcerosa, glomerulonephritis and sepsis.

It is known that INFγ also has antiviral activity. Since the truncated form of INFγ is shown to be bioactive and found to display all activities of native INFγ provided a homing device is attached to the molecule, an analog of the invention is endowed with antiviral activity as well. Exemplary diseases included infections caused by influenza virus or human respiratory syncytial virus (RSV). RSV is a virus that causes respiratory tract infections. It is the major cause of lower respiratory tract infection and hospital visits during infancy and childhood. Sometimes an infant can become symptomatically infected more than once, even within a single RSV season. In the United States, 60% of infants are infected during their first RSV season, and nearly all children will have been infected with the virus by 2-3 years of age. Of those infected with RSV, 2-3% will develop bronchiolitis, necessitating hospitalization. Severe RSV infections have increasingly been found among elderly patients. There is no vaccine. Treatment is limited to supportive care, including oxygen. In temperate climates there is an annual epidemic during the winter months. In tropical climates, infection is most common during the rainy season.

The present disclosure hence also relates to a method for therapeutic or prophylactic treatment of a disease selected from cancer, viral disease, fibrotic disease, sclerotic disease and chronic or acute inflammatory diseases such as glomerulosclerosis, interstitial fibrosis, lung fibrosis, atherosclerosis, rheumatoid arthritis, Crohns disease, colitis ulcerosa, glomerulonephritis and sepsis, comprising providing to a subject in need thereof a therapeutically effective dose of an IFN analog according to the invention. The disease may be a liver disease, preferably a chronic liver disease such as liver cirrhosis. The person skilled in the art will adjust the dosage to be applied to the manner of application, size, weight, state of health etc of the subject to which administration is to occur. Administration can occur in any manner known per se for administration of a medicament. An IFNγ analog according to the invention is advantageously used in a medicinal composition (therapeutic or prophylactic) in a form for intrapulmonary delivery, e.g. by intranasal administration or inhalation. Also provided is an inhalation device comprising an IFNγ analog as active ingredient..

### LEGEND TO THE FIGURES

Figure 1: cloning of mimetic IFNγ from the mouse. Splenocytes were stimulated with PHA and RNA was isolated. RT and PCR using specific primers yielded a PCR product of the expected size. Expression of the construct in BL21 cells and Western blotting confirmed production of the mimetic.

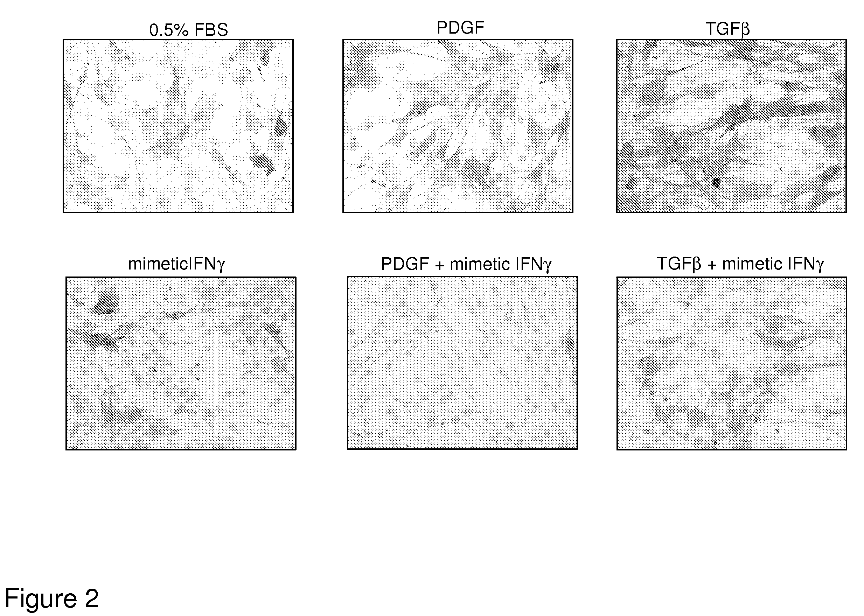

Figure 2: Recombinant mimetic IFNγ shows anti-fibrotic effects in mouse 3T3 fibroblasts. Cells are stained for α-Smooth muscle actin which is a fibrosis marker.
Figure 3: Construction of pET39b-BiPPB encoding a bicyclic PDGF-receptor targeting domain.
Figure 4: Construction of pET39b-BiPPB-IFNgamma encoding a PDGFR-targeted IFNγ conjugate.
Figure 5: Construction of pET39b-BiPPB-mimeticIFNγ.
Figure 6: Dot Blot analysis of IFNγ-BiPPB and Mimetic IFNγ-BiPPB showing the coupling of pPB-peptide to INFγ.
Figure 7: Binding study in human hepatic stellate cell line LX2 cells. Cells were stained for PDGFR-binding peptide. Staining indicates the binding of pPB-containing constructs to the target cells.
Figure 8: Blood count analysis to study side effects of the treatment in acute liver injury mouse model. WBC= white blood cell count. LYM= lymphocytes. PLT= platelet count. RBC= red blood cell count. For details see Example 4.
Figure 9: Real Time PCR analysis of the liver fibrosis markers MMP13 and TIMP1. For details see Example 4.
Figure 10: Effect of mimetic IFN□-PEG-BiPPB in the acute CCl₄-induced liver-injury mouse model. Quantitation of a-SMA (A), collagen-I (B) and desmin (C) - staining of liver sections obtained from olive oil-treated normal mice and CCl₄-treated mice receiving either IFNγ, mimetic IFNγ, mimetic IFNγ-PEG, mimetic IFNγ-PEG-BiPPB (5 µg/mice) or PBS alone. Quantitation was done using computerized Cell-D imaging software. Data represent the mean ± SEM from 5 mice per group. #P<0.05 versus PBS-treated olive oil group; *P<0.05, **P<0.01 versus PBS treated-CCl₄ group. For details see Example 6
Figure 11: Real Time PCR analysis of markers for liver fibrosis in mice, 3 days after CCl4 administration or olive oil. Mice were treated with different compounds as indicated and Collagen 1a1 (A), a-SMA (B), Desmin (C) and TIMP1 (D) mRNA levels were measured. For details see Example 6.
Figure 12: Blood count analysis in whole blood of mice, 3 days after CCl4 administration or olive oil. Mice were treated with different compounds as indicated and Platelet count (PLT: fig A), white blood cell count (WBC, fig B) and lymphocyte count (C) were measured using a coulter counter. For details see Example 6.

### EXPERIMENTAL SECTION

Liver Fibrosis is characterized by the excessive accumulation of the extracellular matrix components that leads to hepatic scars. To date, no successful therapy is available for the treatment of liver fibrosis. Hepatic stellate cells (HSCs) and fibroblasts are the key effector cells involved in the progression of the disease, which are activated by crucial growth factors like Platelet derived growth factor (PDGF) and transforming growth factor-beta (TGFβ). Interferon gamma (IFNγ) has been shown to have various beneficial effects *in-vitro* and *in-vivo* during liver fibrosis. However, IFNγ displays a strict species specificity and has a short circulating half life which limits its potential clinical use. Moreover, INFγ has serious adverse effects on the immune system, on endothelial cells and on the Central Nervous System (e.g. causing depressions) that all lead to frequent withdrawal of patients from clinical trials and consequently to failure of these trails. To circumvent these drawbacks, the present inventors produced a stable peptide mimetic of IFNγ that lacks the extracellular receptor recognition site and contains a signalling moiety which interacts directly at the downstream IFNγ signalling cascade, thereby retaining the prospective functions of IFNγ.

To increase the specificity of IFNγ and IFNγ mimetic peptide, IFNγ and mimetic peptide fused to BiPPB (**Bi**cyclic **p**eptide against the **P**DGF-**b**eta -receptor) were been generated, since PDGF receptor expression is highly up-regulated during liver injury particularly in HSCs.

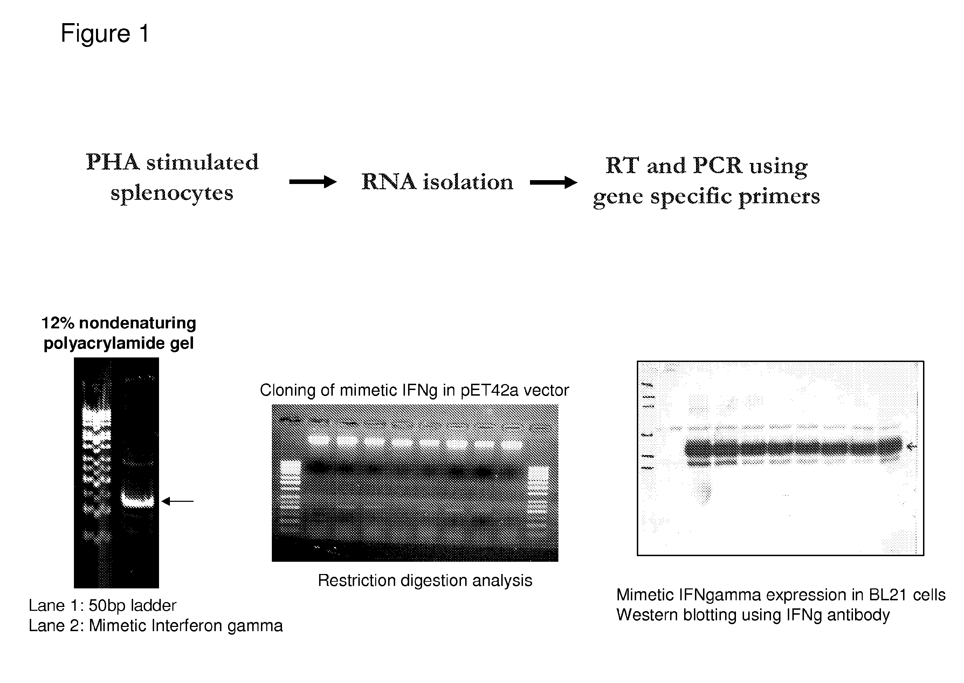

**Example 1: Cloning of mimetic IFNγ** Mouse splenocytes were isolated from fresh spleens and were cultured in the presence of PHA (Phytohemagglutinin) to stimulate cytokine production. After 24 hrs of stimulation, RNA was isolated and cDNA was synthesised using gene specific reverse primer followed by PCR amplification by phusion DNA polymerase using mimetic IFNγ specific forward and reverse primers. The obtained fragment was cloned in pET42a (prokaryotic expression vector) at PshAl/EcoRI site and the positive clones were checked by restriction digestion analysis. See Figure 1.

The 5'-> 3' nucleotide sequence of the truncated mouse Interferon gamma (NCBI Reference sequence: NM_008337.2) (nt 457 - nt 572) is as follows:
457 gcca agtttgaggt caacaaccca caggtccagc gccaagcatt caatgagctc atccgagtgg tccaccagct gttgccggaa tccagcctca ggaagcggaa aaggagtcgc tg 572 a The last nucleotide has been added in order to insert the stop codon before cysteine,
which is the last amino acid in the sequence. Cysteine is removed from the sequence to provide appropriate folding of the peptide and also to avoid inappropriate folding due to disulfide bonds in the fusion protein (with BiPPB).

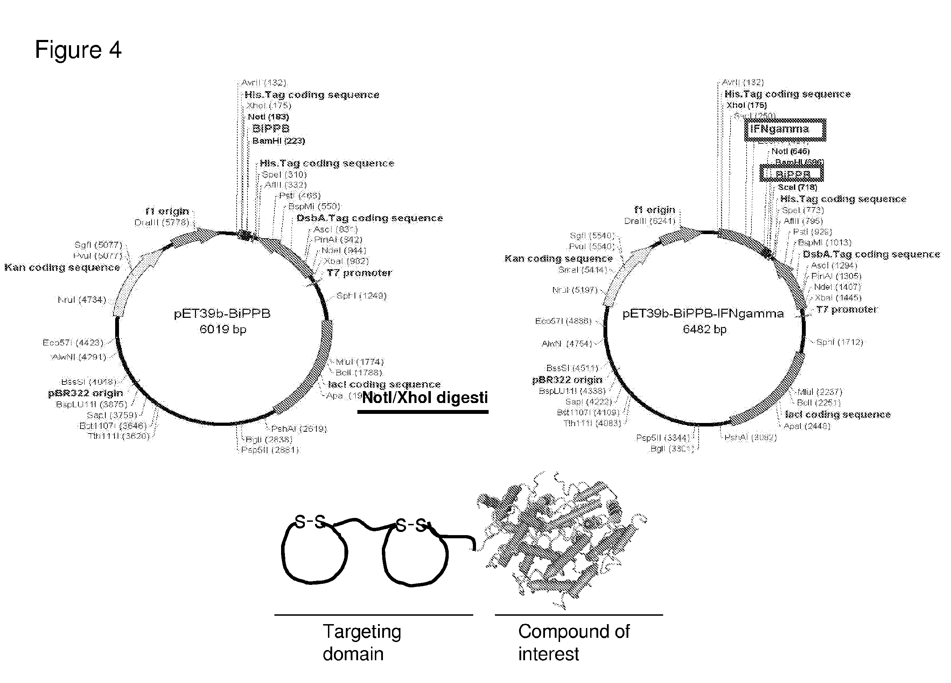

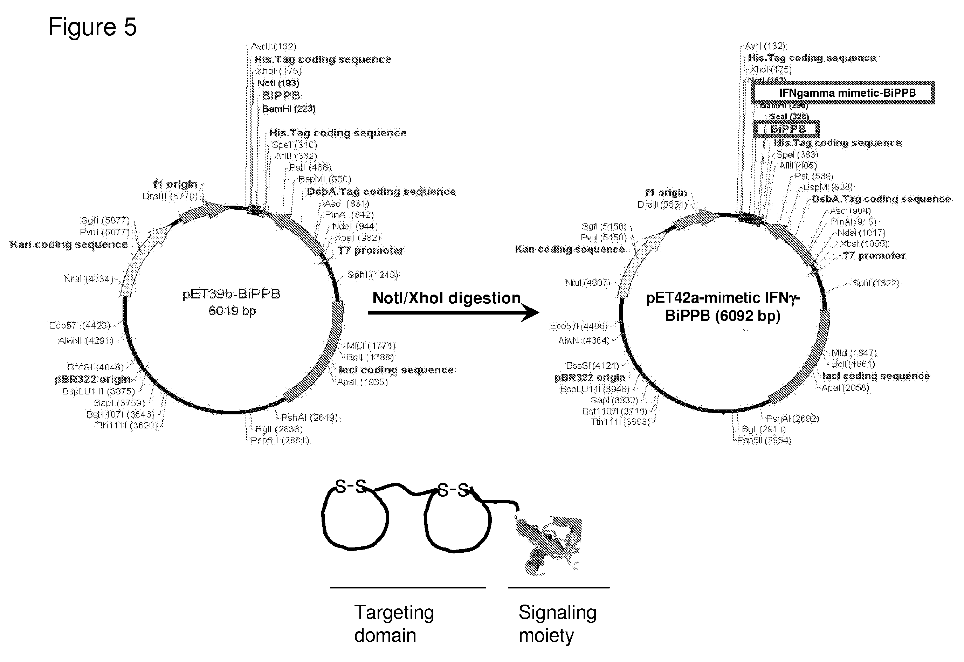

The encoded amino acid sequence is AKFEVNNPQVQRQAFNELIRVVHQLLPESSLRKRKRSR* * Denotes Stop codon
**Cloning of IFNγ-BiPPB and mimetic IFNγ-BiPPB** A nucleic acid sequence encoding the bicyclic PDGF targeting domain BiPPB was generated by amplification of two fragments using 4 primers (2 for each fragment) and then ligated using inbuilt Bam HI restriction site and was then cloned in pET39b vector at ScaI/NotI site. IFNγ and Mimetic IFNγ was PCR amplified using peptide fusion primer (forward) and IFNγ or mimetic IFNγ reverse primer. The amplified fragment was digested and ligated in pET39b-BiPPB vector at NotI/XhoI site and the positive clones were checked by restriction digestion analysis. See Figures 4 and 5. The sequences of all the constructs were further confirmed by automated DNA sequencing.

### BiPPB

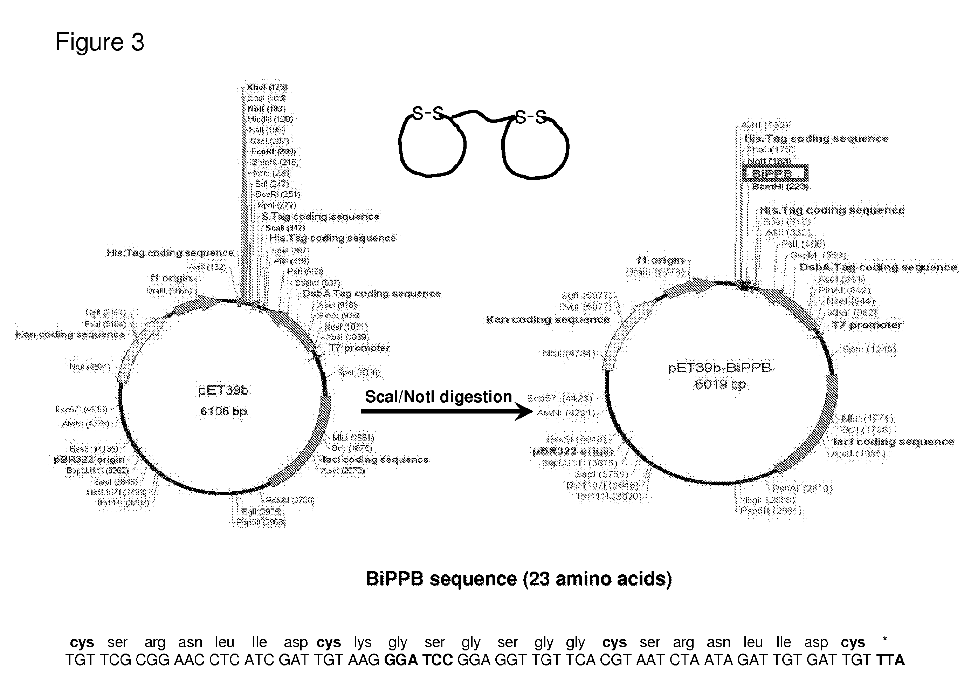

Nucleotide sequence for BiPPB:
tgt tct aga aac ctc atc gat tgt aag gga tcc gga ggt tgt tca cgt aat cta ata gat tgt tca Amino acid sequence for BiPPB: CSRNLIDCKGSGGCSRNLIDCS (see also Figure 3)

### Interferon gamma (full length)

Nucleotide sequence: mouse Interferon gamma (NCBI Reference sequence: NM_008337.3) gcggccgca 457 gcca agtttgaggt caacaaccca 481 caggtccagc gccaagcatt caatgagctc atccgagtgg tccaccagct gttgccggaa 541 tccagcctca ggaagcggaa aaggagtcg 569 ataa Amino acid sequence for Mouse IFNgamma

MNATHCILALQLFLMAVSGCYCHGTVIESLESLNNYFNSSGIDVEEKSLFLDIWRN
WQKDGDMKILQSQIISFYLRLFEVLKDNQAISNNISVIESHLITTFFSNSKAKKDAF
MSIAKFEVNNPQVQRQAFNELIRVVHQLLPESSLRKRKRSRC

Nucleotide sequence of the fused protein (BiPPB-IFN gamma) tgt tct aga aac ctc atc gat tgt aag gga tcc gga ggt tgt tca cgt aat cta ata gat tgt tca gcggccgca Interferon gamma sequence (NM_008337.3).

Amino acid sequence for BiPPB-IFNgamma

CSRNLIDCKGSGSGGCSRNLIDCSAAAMNATHCILALQLFLMAVSGCYCHGTVIES
LESLNNYFNSSGIDVEEKSLFLDIWRNWQKDGDMKILQSQIISFYLRLFEVLKDNQ
AISNNISVIESHLITTFFSNSKAKKDAFMSIAKFEVNNPQVQRQAFNELIRVVHQLL
PESSLRKRKRSRC

Italics denotes BiPPB; normal text denotes IFNgamma mimetic; Bold denotes linker or spacer. See also Figure 4.

### Mimetic Interferon gamma fused to BiPPB

Nucleotide sequence of the fused protein (BiPPB-IFN gamma mimetic) tgt tct aga aac ctc atc gat tgt aag gga tcc gga ggt tgt tca cgt aat cta ata gat tgt tca gcggccgca 457 gcca agtttgaggt caacaaccca caggtccagc gccaagcatt caatgagctc atccgagtgg tccaccagct gttgccggaa tccagcctca ggaagcggaa aaggagtcg 569 ataa
Amino acid sequence for BiPPB-IFNgamma mimetic

CSRNLIDCKGSGSGGCSRNLIDCSAAA
AKFEVNNPQVQRQAFNELIRVVHQLLPESSLRKRKRSR*

Italics denotes BiPPB; normal text denotes IFNgamma mimetic; Bold denotes: linker or spacer. See also Figure 5.

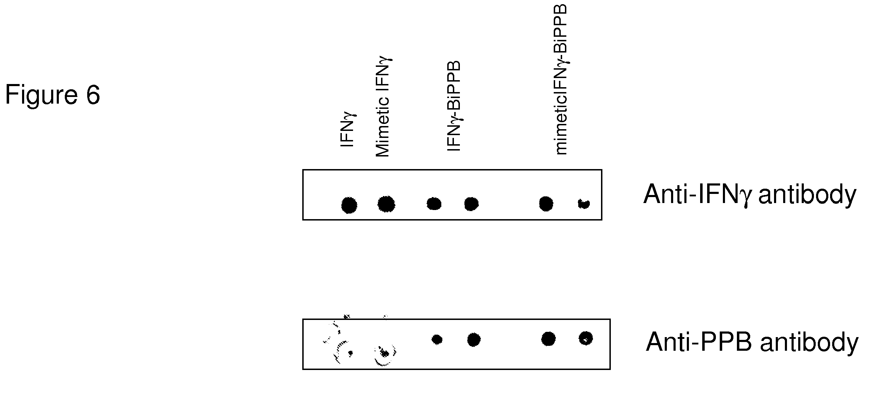

The nucleic acids were then transformed into BL21 cells *(E. coli*) for the expression using IPTG induction. The expressed protein was analysed by SDS-PAGE and Western blot analysis or Dot Blot analysis using anti-IFNγ antibody and/or anti-PPB antibody (see Figure 6). The expressed protein (with His Tag) was then purified under native conditions through Ni-NTA column chromatography. The tags were proteolytically cleaved and further purified using sepharose column chromatography.

**Example 2: Anti-fibrotic effects of cleaved and active mimetic IFNγ:** We evaluated the anti-fibrotic effects of cleaved IFNγ mimetic peptide in mouse NIH3T3 fibroblasts as assessed by immuno-cytochemistry (α-SMA staining). Briefly, 3T3 fibroblasts were seeded in 24 well plates at the density of 6 x 10⁴ cells/well, After 24 hrs, cells were starved in 0.5% FBS containing medium for overnight. Thereafter, cells were incubated in starvation medium with different compounds (PDGF 50 ng/ml, TGFβ 10 ng/ml, mimetic IFNγ 1µg/ml, 50ng/ml PDGF + 1µg/ml mimetic IFNγ and 10 ng/ml TGFβ + 1µg/ml mimetic IFNγ). After 48 hrs of incubation, cells were washed, fixed with ethanol:acetone (1:1) and stained for α-SMA (marker of activated fibroblasts).

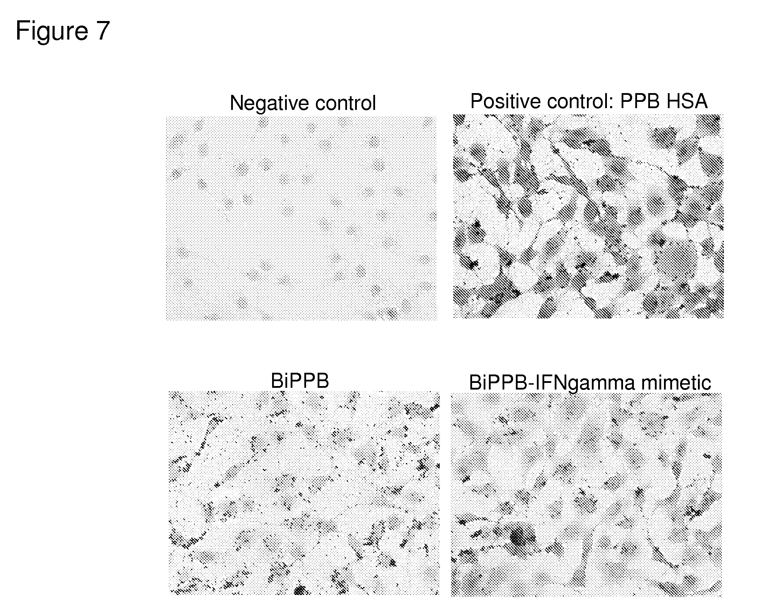

**Example 3: Binding study of BiPPB and mimetic IFNγ-BiPPB in human LX2 cells:** We determined the binding of BiPPB and mimetic-BiPPB in LX2 cells (human HSCs). Briefly, LX2 cells were plated in 48 well plates at the cell density of 3 x 10⁴ cells/well. After 24 hrs, cells were starved in medium (-FBS) for overnight. Then, cells were incubated with different compounds (BiPPB, PPB-HSA and BiPPB-Mimetic IFNγ) for 2 hrs at room temperature for binding. After binding, cells were extensively washed with PBS, fixed with ethanol:acetone (1:1) and stained for PPB. Results are presented in Figure 7.

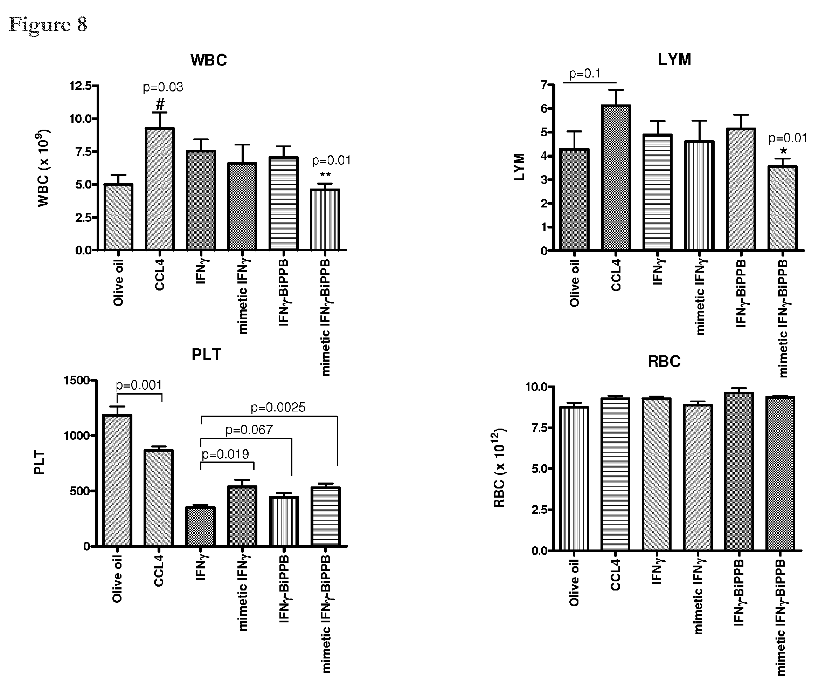

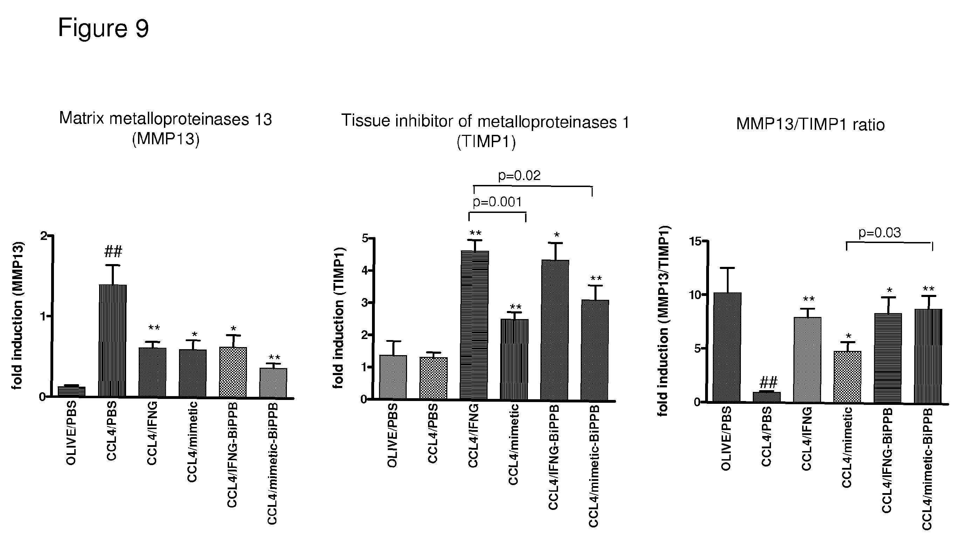

**Example 4: In-vivo effect study in acute CCl₄-induced liver injury in mice:** Recombinant IFNγ, mimetic IFNγ, recombinant fusion protein IFNγ-BiPPB and recombinant fusion protein mimetic IFNγ-BiPPB were tested for anti-fibrotic effects in acute CCL₄-induced liver injury mouse model. At day 1, the animals were given a single intra-peritoneal dose (1ml/kg) of carbon tetrachloride (CCl₄) in olive oil or olive oil (controls n=6). After 24 hrs of CCl₄ injection, at day 2 and 3, animals were treated either with PBS (n=6), 50,000 U/mice of IFNγ (n=6), 50,000 U/mice of mimetic IFNγ (n=5), 50,000 U/mice of IFNγ-BiPPB (n=6), 50,000 U/mice of Mimetic IFNγ-BiPPB (n=6). Thereafter, at day 4, animals were sacrificed and blood counts were performed and anti-fibrotic effects (See Hemmann S, Graf J, Roderfeld M, Roeb. J Hepatol. 2007 May;46(5):955-75) were evaluated using quantitative PCR. Results are presented in Figures 8 and 9.

The data presented in Fig 8. demonstrate that mimetic-BiPPB is more effective than unmodified INFγ in attenuating the upregulation of white blood cell count (WBC) and lymphocyte count (lym) in whole blood associated with CCl₄-induced liver fibrosis (p<0.01). In contrast, the reduction in platelet count (PLT) associated with INFγ treatment (which is a well known adverse effect of INFγ) is less severe when animals receive mimetic-INFγ-BiPPB instead of unmodified INFγ (p< 0.0025).

The data presented in Figure 9 show that mimetic-INFγ-Bi-PPB is endowed with antifibrotic activity: it attenuates CCl₄-induced upregulation of matrix metalloproteinases (MMP13). The ratio MMP-13 versus Tissue Inhibitor of Metalloproteinases (TIPM1) is similar to that obtained with native INFγ, but better than mimetic INFγ (p<0.03), indicating that coupling of Bi-PPB to mimetic INFγ is beneficial. Collectively the data in Fig. 8 and 9 show that mimetic INFγ-BiPPB is more potent compared to native INFγ and has less side effects.

### EXAMPLE 5:

**Chemical synthesis of Mimetic Interferon gamma conjugates:** Mimetic IFNγ-PEG-BiPPB conjugate: 0.111 µmol Bicyclic PDGFR recognizing peptide (BiPPB, 2223.2 Da, Genosphere Biotechnologies) was coupled with 0.337 µmol maleimide-PEG-succinimidyl carboxy methyl ester (Mal-PEG-SCM, 2 KDa, Creative PEGworks) for 3 hrs. Thereafter, lysine (0.337 µmol) was added to block free groups of Mal-PEG-SCM. After 1 hr of reaction, the synthesized product BiPPB-PEG-MAL (0.112 µmol) was reacted with 0.56 µmol of Mimetic IFNγ-ATA (4689 Da, Genosphere Biotechnologies) in the presence of deacetylating reagent for overnight at room temperature. Finally, the synthesized Mimetic IFNγ-PEG-BiPPB conjugate (8828.2 Da) was extensively dialyzed against PBS using 7 KDa slide-α-lyzer G2 dialysis cassettes (Thermo scientific).

Mimetic IFNγ-PEG conjugate: 0.107 µmol Mimetic IFNγ-ATA (4689 Da) was reacted with 0.321 µmol of Poly (ethylene glycol)-succinimidyl α-methylbutanoate (mPEG-SMB, 2 KDa, Nektar therapeutics) for 2 hrs and subsequently the product was dialyzed extensively.

### a) Mimetic IFNγ-PEG-BiPPB

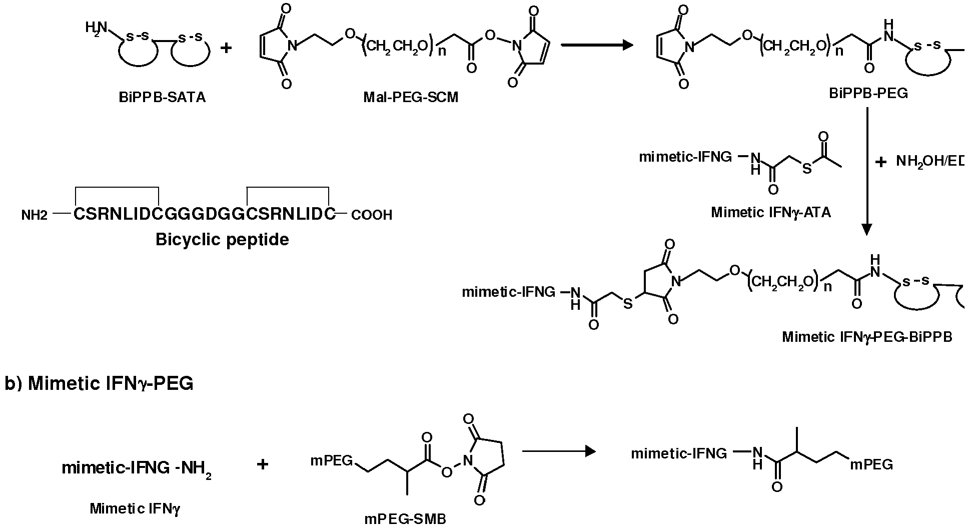

### EXAMPLE 6:

### Effect on fibrotic parameters after intravenous administration of mimeticIFNγ-PEG-BiPPB in acute liver injury mouse model

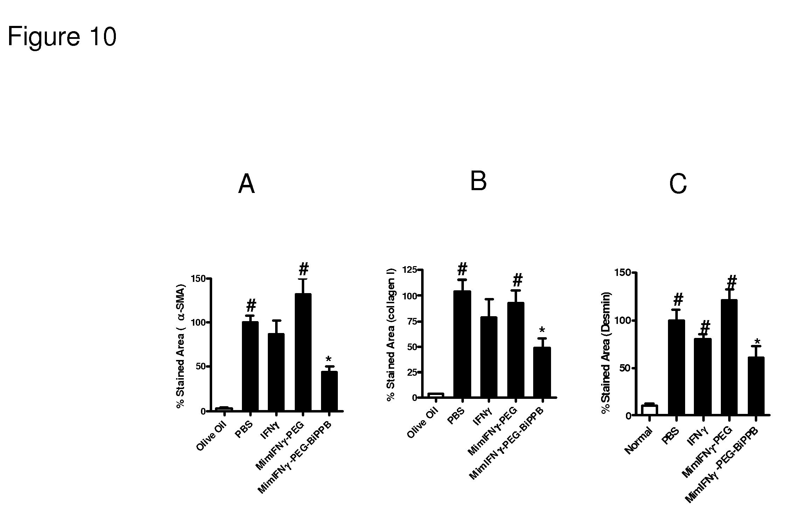

Analysis of fibrotic parameters at the protein level:
Mice were intraperitoneally injected with CCl₄ at day 1 to induce liver injury. At day 2 and 3, mice were treated with IFNγ (5 ug/dose), mimetic IFNγ-PEG, mimetic IFNγ-PEG-BiPPB (5µg/dose) or PBS alone. At day 4, animals were sacrificed; livers and different organs were collected for further analysis. Liver-sections were fixed with acetone, dried and rehydrated with PBS. Then, the sections were incubated with primary antibody (collagen, SMA and Desmin) for 1 hr. Thereafter, the sections were blocked with 0.03% H₂O₂ for endogenous peroxidase activity for 30 min. Subsequently, sections were incubated with secondary antibody HRP conjugated rabbit anti-goat antibody (1:100, DAKO) followed by HRP conjugated goat anti-rabbit antibody (1:100, DAKO) for 30 min. The peroxidase activity was developed using AEC (Sigma) for 20 min and nuclei were counterstained with hematoxylin (Fluka). The sections were mounted with Kaiser's gelatin and visualized under the light microscope (Olympus). For quantitative analysis, 27 microscopic pictures were captured and positively-stained areas were quantified using computerized Olympus Cell D imaging software. Results are shown in Figure 10

### Analysis of fibrotic parameters at the gene expression level:

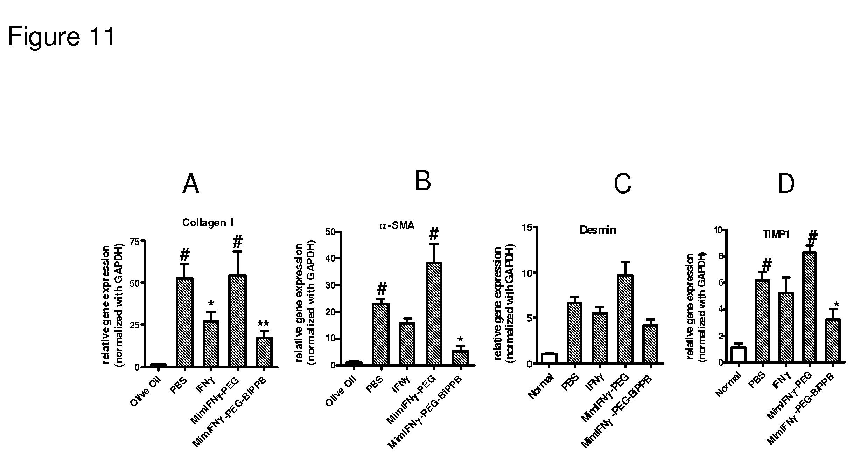

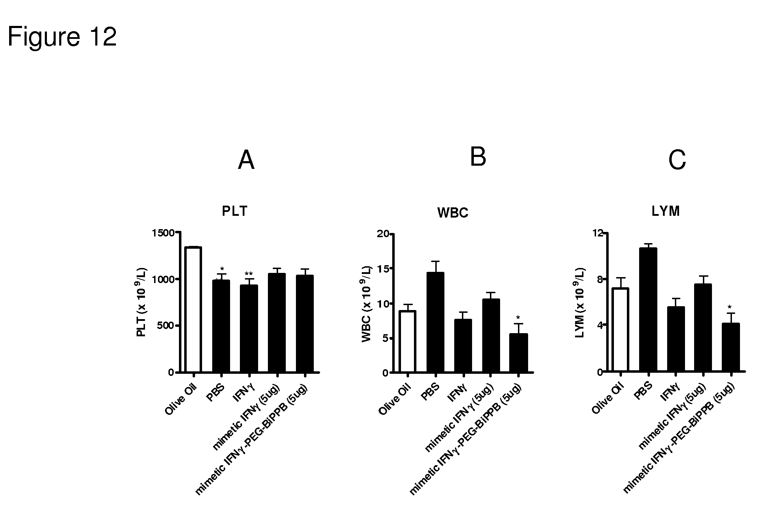

Total RNA from liver tissues was isolated using RNeasy mini kit (Qiagen) according to the manufacturer's instructions. The RNA concentration was quantitated by a UV spectrophotometer (NanoDrop Technologies, Wilmington, DE). Total RNA (1.6 µg) were used for reverse transcription in total volume of 50 µl with the cDNA synthesis kit (Promega). All primers were purchased from Sigma-Genosys (Haverhill, UK). 10 ng of cDNA was used for quantitative real time PCR analysis. The reactions were performed using SYBR green PCR mix (Applied Biosystems) according to manufacturer's instructions. The samples were analyzed by ABI 7900HT sequence detection system (Applied Biosystems). Finally, the threshold cycle numbers (Ct) were calculated for each gene and relative gene expression was calculated after normalizing for expression of the reference gene GAPDH. Results are shown in Figure 11. Analysis of adverse effects is depicted in Figure 12.

### EXAMPLE 7:

### Effect on fibrotic parameters after intravenous administration of mimeticIFNγ-PEG-BiPPB in established advanced liver fibrosis mouse model

### Analysis of fibrotic parameters at the protein level:

Male balb/c mice (20-22g) were treated with olive oil or increasing doses of CCl₄ (week 1: 0.5 ml/kg; week 2: 0.8 ml/kg and week 3-8: 1 ml/kg prepared in olive oil) twice weekly by intra-peritoneal injections for 8 weeks. In week 7 and 8, mice were treated intravenously with PBS, mimeticIFNγ-PEG or MimeticIFNγ-PEG-BiPPB (5µg/mice, thrice per week). All mice were sacrificed at week 8; blood and liver samples were collected for subsequent measurements. The liver sections were stained for Collagen I and desmin, CD68, 33D1 and MHC class II. It was found that the targeted truncated form of IFNγ (mimIFNγ-biPPB) induced substantial reduction in the fibrotic parameters in this chronic liver fibrosis model in mice; both collagen I and desmin staining was profoundly reduced in mimIFNγ-biPPB -treated CCl4 mice compared to untretaed CCl4-mice. In contrast, treatment with untargeted mimIFNγ, lacking the receptor binding site, induced no effect on these parameters while full length mouse IFNγ induced only a small reduction in collagen I and desmin staining. The native (mouse) IFNγ induced infiltration of inflammatory cells (CD68⁺ macrophages, neutrophils, 33D1⁺ dendritic cells) as well as increased MHCII expression. In contrast, MimIFNγ-BiPPB did not induce this increased inflammatory response in livers.

### EXAMPLE 8 :

### Truncated IFNγ analog targeted to the PDGF receptor is active but causes less side effects.

A targeted conjugate of BiPPB chemically coupled mimetic-IFNγ was synthesized and characterized using Western blot analyses and for its anti-fibrotic effects *in vitro* in mouse 3T3 fibroblasts. In vivo, the targeted conjugate was examined in 4 days (acute) and 8 weeks (chronic) liver fibrosis models induced with CCl₄ in mice. Several fibrotic parameters and infiltration of inflammatory cells were assessed in the livers using immunohistochemistry and gene expression analysis.

**Results:** The successfully synthesized conjugate caused inhibition of collagen expression in TGFbeta-induced mouse fibroblasts. *In vivo,* the targeted peptidomimetic of IFNγ (mimIFNγ-biPPB) induced substantial reduction in the fibrotic parameters in both acute and chronic liver fibrosis models in mice. Treatment with untargeted mimIFNγ, which lacks a receptor binding domain, showed no effect and unmodified mouse full length IFNγ showed only a moderate reduction. This mouse full length IFNγ induced infiltration of inflammatory cells (CD68⁺ macrophages, neutrophils, 33D1⁺ dendritic cells) as well as increased MHCII expression. In contrast, MimIFNγ-BiPPB did not induce an inflammatory response (data not shown).

### EXAMPLE 9:

### Study with MimeticIFNγ-PEG-BiPPB in subcutaneous tumor model in mice

### Materials and Methods

Normal male C57BL/6 and Balb/c mice weighing 20 to 25 g were obtained from Harlan (Zeist, the Netherlands). They were kept at a 12:12-hour light/dark cycle and received ad libitum normal diet. All experimental protocols for animal studies were approved by the Animal Ethics Committee of the University of Groningen. To induce subcutaneous tumors, C26 cells were cultured in 125-mm³ flasks a day before injection in animals to keep them in the growth phase. Cells were detached by trypsanization, and trypsin was removed by centrifugation. The cell pellet was resuspended in PBS. A total of 1 × 10⁶ cells (B16 and C26 cells) suspended in 100 µl of PBS were injected subcutaneously in the flank of Balb/c mice. Tumor growth was followed by measuring tumor size using a digital Vernier caliper. Tumor volume was established using the formula: a × b2 / 2, where a denotes tumor length and b denotes the tumor width. C26 tumors were induced in mice as described. The treatment was started on day 5 when the tumor volume was reached the range of 50 to 100 mm³ because this tumor size has been shown as an optimum tumor size for the start of the treatment. Animals (n=4 per group) were injected intravenously with six doses of either vehicle (PBS), mimeticIFNγ-PEG (5 µg/dose), mimeticIFNγ-PEG-BiPPB (5 µg/dose) on alternative days under anesthesia (O₂/isoflurane). Tumor size was measured under anesthesia. The animals with C26 tumors were killed on day 20 because no effect of the treatment was observed. Animals were killed under gas anesthesia (O₂/isoflurane), and tumors were isolated and fixed in cold isopentane for cryosections.

4-µm-thick cryostat sections were prepared from snap-frozen tissue and stained for CD31 according to standard immunoperoxidase methods. We analyzed CD31 staining (endothelial cell marker) for the determination of the blood vessel lumen area and blood vessel density in tumor sections of C26 tumors. Results showed significant angiogenesis in untreated tumors and in tumors of mice treated with mimetic IFNγ, while mice treated with mimetic IFNγ-BiPPB displayed a strong reduction in angiogenesis in their tumors (data not shown).

### EXAMPLE 10:

### Effect of PDGF-receptor targeted truncated INFγ on pulmonary fibrosis.

### Background and rationale for INFγ-based therapies in IPF patients:

Idiopathic pulmonary fibrosis (IPF) is a progressive parenchymal lung disease with a median survival of only 3-5 years following diagnosis^{1,2}. Besides IPF also other types of lung fibrosis have a poor prognosis, in particular fibrosing non-specific interstitial pneumonia (NSIP) and end-stage fibrosis of for example several auto-immune diseases and extrinsic allergic alveolitis. There are currently no effective therapies for lung fibrosis due to poor understanding of the disease mechanisms.

The (myo)fibroblast has a central role in all types of lung fibrosis³. Ongoing damage to the lungs leads to initiation of dysregulated repair mechanisms with recruitment of fibroblasts and their transformation to myofibroblasts. Myofibroblasts are the key effector cells in fibrosis producing excessive amounts of extracellular matrix components like collagens. Myofibroblasts are currently thought to be the most important therapeutic target for treatment of lung fibrosis³. Key growth factors in the proliferation and transformation of myofibroblasts are transforming growth factor beta (TGFβ), platelet-derived growth factor (PDGF), and endothelin-1 and many new therapies have focussed on inhibiting these factors or their receptors. However, most of the clinical trials based on modulating myofibroblast behaviour have shown disappointing results¹.
Interferon gamma (IFNγ) is probably the most-studied anti-fibrotic mediator in clinical trials of IPF. It mediates myofibroblast growth arrest and apoptosis through a STAT-1 (Signal Transducers and Activators of Transcription-1)-dependent pathway, which is important for resolution of fibrogenic responses⁴. Despite an initial promising start, the large INSPIRE trial concluded that IFNγ did not prolong survival⁵. IFNγ, however, was administered subcutaneously and since IFNγ receptors are found on almost all cells in the body, it has severe dose-limiting side effects. These include influenza-like illness and fatique⁵. Inhaled administration can partly circumvent this problem and one trial is currently registered to study the effects of aerosolized IFNγ in IPF (see http://clinicaltrials.gov).
Another way to increase the concentration of IFNγ within myofibroblasts, and thus the efficacy, is to use the concept of drug targeting: drugs are coupled to cell-selective drug carriers that are specifically taken up by target cells, the drugs are released within those cells thereby reducing systemic side effects while attaining high local concentrations in target cells. Myofibroblasts for instance specifically upregulate PDGF-receptors that can be used for drug targeting purposes⁶.

Although CCl₄ induces liver fibrosis, the lungs also have low expression of Cytochrome P450 activity and CCL₄ (turned into toxic compounds by this enzyme) is therefore also slightly activated in lung tissue. These leads to activation of profibrotic cells which in turn express the PDGF receptor, which initiates profibrotic activity. This model has been used to as a model of idiopathic pulmonary fibrosis (IPF, see ref 8 and 9) . While exploring the antifibrotic effects of INFγ in liver tissue, a significant change in lung tissue was noted by the examiners. The weight of lungs from CCl₄-treated mice was significantly higher than lungs of normal mice (0.75 ± 0.05 % of body weight in normal versus 2.03 ± 0.15 % of b.w. in untreated CCl4-treated mice; P< 0.01) yet this increase was significantly reduced by treatment with PPB-PEG-INFγ (1.53 ± 0.11 % of b.w.; P<0.05 vs untreated CCl4 mice). Microscopical examination revealed that the lungs of CCl₄-treated mice were affected by diffuse alveolitis, a condition which can precede fibrosis, and lungs displayed an enhanced collagen staining. When these mice were treated with IFNγ coupled to PDGF-receptor recognizing peptides, we found a significantly reduced alveolitis in lungs and a reduced collagen deposition. Of note, these beneficiary effects of targeted IFNγ were attained *after* establishing the lung disease.

We therefore conclude that PDGF-receptor-targeted INFγ is able to accumulate in any tissue with significant PDGFβ-receptor expression, and is able to exert an antifibrotic effect in other tissues as well as illustrated by its effect in lungs. The INFγ analogs of the invention may thus be used for the treatment of idiopathic fibrosis and other forms of fibrosis or sclerosis in other tissues characterized by enhanced expression of the PDGF-receptor.

### EXAMPLE 11:

### Targeting of truncated IFNgamma to various receptors

In this experiment it is shown that an analog of the invention comprising the signaling moiety of murine IFNγ (mimIFNγ) and a cell surface targeting domain can be efficiently targeted not only to the PDGF receptor but also to other cell surface receptors. Exemplary targeting domains tested include lactose (ligand for the Asialoglycoprotein (ASGP)), mannose (ligand for the mannose receptor (CD 206) and the tripeptide RGD (ligand for the receptor αvβ3 integrin receptor). The sequence of mimIFNγ consisted of FEVNNPQVQRQAFNELIRVVHQLLPESSLRKRKRSR. Various cell types and different parameters for testing IFNγ activity used in the study. The control samples were exposed to intact murine INNγ. For details see the Table below.

| **IFNγ analog** | **Target receptor** | **Cells used** | **Parameter measured (details see text)** | **Results** |
|---|---|---|---|---|
| Lactose-HSA-PEG-mimIFNγ | Asialoglycoprotein (ASGP) receptor | Human Hepatocytes (HepG2) | ICAM 1 expression | enhanced expresion vs control INFγ |
| Mannose-HSA-PEG-mimIFNγ | mannose receptor (CD 206) | Mouse RAW macrophages | MHC II expression | equal expression vs control INFγ |
| RGD-PEG-mimIFNγ | αvβ3 -receptor | Endothelial cells | *In vitro* angiogenesis assay | Enhanced effectivity vs control INFγ |

### Experiments with Lactose-HSA-PEG-IFNγ.

### Synthesis

Lactose-HSA (25 lactose molecules coupled to human serum albumin) was conjugated to bifunctional PEG molecule (2KDa). Following dialysis, lactose-HSA-PEG was coupled to SATA-modified truncated IFNγ derived from mouse. The synthesized product (Lac-HSA-PEG-mimIFNγ) was extensively dialysed against PBS.

### Experiment in human Hepatocytes:

Human Hepatocytes (HepG2) were plated in 12 well plates (1 x 10⁵ cells/well). The cells were grown overnight in 5%/37°C/CO₂ incubator. Subsequently, cells were incubated with medium, mouse IFNγ (1ug/ml), Human IFNγ (1ug/ml), mouse IFNγ derived Lac-HSA-PEG-IFNγ (1ug/ml), Lac-HSA or Lac-HSA-PEG-IFNγ (1ug/ml) after 2 hrs of blocking with Lac-HSA. After 24 hrs of incubation, cells were lysed, RNA was isolated and reverse transcribed. The cDNA was used for the analysis of InterCellular Adhesion Molecule 1 (ICAM1) expression, which is known to be induced in response to IFNγ. 18srRNA was used a housekeeping control.

### Results:

As expected, neither mouse truncated IFNγ nor Lac-HSA induced ICAM1 expression in human hepatocytes, while both human IFNγ and mouse derived Lac-HSA-PEG-IFNγ upregulated ICAM1 expression in human hepatocytes. Furthermore, Lac-HSA-PEG-IFNγ-induced ICAM1 expression was almost completely blocked by excess of Lac-HSA.

### Conclusions:

The species-specificity of INFγ was confirmed by showing that mouse (truncated) INFγ was ineffective in human hepatocytes. However, this cytokine was turned into a bioactive compound in human cells after coupling to the target moiety lactose, which is known to enter hepatocytes via the Asialoglycoprotein (ASGP) receptor. Specificity for the ASGP-receptor was shown by blockade of this receptor by Lac-HSA. This demonstrates that the signaling part of INFγ (which is not species specific) can be delivered into the cytoplasm of other target cells than fibroblasts via another target receptor than the PDGF-receptor using a sugar moiety instead of a peptide. Hepatocyte targeting is particularly relevant for the treatment of Hepatitis B and C and ICAM-1 upregulation is a physiological response to enhance antiviral activity. This experiment supports the use of cell-specific- truncated INFγ according to the invention as an antiviral compound.

Similarly, mannose was coupled to truncated mouse INFγ according to standard techniques (L. Beljaars et al J. of Hepatology 29: 579-588, 1998) and bioactivity of this mannosylated INFγ was demonstrated in mouse macrophages with MHC class II expression as read-out parameters (rtPCR methods).

The endothelial binding peptide RGD- or its control peptide RAG, that does not bind to these cells, were also coupled to truncated mouse INFγ according to standard methods. The resulting analogs were evaluated for their biological activity in cultures of endothelial cells (H5V) by measuring tube formation which is a parameter reflecting angiogenesis. Tube formation by H5V cells was inhibited most potently by RGD-mimINFγ in this assay (data not shown).

Conclusions: Modification of truncated mouse INFγ by conjugation to a targeting moiety (e.g. an oligosaccharide or peptide) is feasible without disturbing the bioactivity of the signaling part of this cytokine. Delivery of an INFγ analog which is defective in binding to its natural receptor but which instead can bind to a distinct cell surface receptor mediating cellular uptake of the analog allows for more effective therapeutic applications with less side-effects.

### References

1. du Bois RM. Strategies for treating idiopathic pulmonary fibrosis. Nat Rev Drug Discov;9(2):129-40.
2. Wilson MS, Wynn TA. Pulmonary fibrosis: pathogenesis, etiology and regulation. Mucosal Immunol 2009;2(2):103-21.
3. Scotton CJ, Chambers RC. Molecular targets in pulmonary fibrosis: the myofibroblast in focus. Chest 2007;132(4):1311-21.
4. Bonner JC. Mesenchymal cell survival in airway and interstitial pulmonary fibrosis. Fibrogenesis Tissue Repair;3:15.
5. King TE, Jr., Albera C, Bradford WZ, et al. Effect of interferon gamma-1b on survival in patients with idiopathic pulmonary fibrosis (INSPIRE): a multicentre, randomised, placebo-controlled trial. Lancet 2009;374(9685):222-8.
6. Beljaars L, Weert B, Geerts A, Meijer DK, Poelstra K. The preferential homing of a platelet derived growth factor receptor-recognizing macromolecule to fibroblast-like cells in fibrotic tissue. Biochemical pharmacology 2003;66(7):1307-17.
7. Homma S, Nagaoka I, Abe H, et al. Localization of platelet-derived growth factor and insulin-like growth factor I in the fibrotic lung. Am J Respir Crit Care Med 1995;152(6 Pt 1):2084-9.
8. Paakko P, Anttila S, Sormunen R, et al. Biochemical and morphological characterization of carbon tetrachloride-induced lung fibrosis in rats. Arch Toxicol 1996;70(9):540-52.
9. Mizuguchi S, Takemura S, Minamiyama Y, et al. S-allyl cysteine attenuated CCl4-induced oxidative stress and pulmonary fibrosis in rats. Biofactors 2006;26(1):81-92.

### EXAMPLE 11:

### Binding study of BiPPB to cultured or freshly isolated primary cells

This example shows that Bi-PPB either produced chemically or through recombinant techniques specifically binds to the PDGFB-receptor. Receptor specificity is demonstrated by blocking the binding with specific antiPDGF-β-receptor antibodies. BiPPB is species non-specific as it binds to Myo-fibroblast-like cells of rat, mouse and human. Receptor interaction requires at least two cyclic peptides, as the monocyclic form does not bind to the target receptor.

### Sequence of BiPPB:

C(1)SRNLIDC(1)GGGDGGC(2)SRNLIDC(2): Cys(1)-Cys(1) and Cys(2)-Cys(2) disulfide bridge cyclisations

### Methods:

The binding of BiPPB was performed in primary freshly isolated rat hepatic stellate cells. Cells were seeded in the 8-well glass plates (Lab-Tek, Nunc, Naperville, IL) at 30,000 cells/well in the culture medium. After overnight incubation at 37°C/5%CO2, cells were washed with PBS and subsequently incubated with FITC-labeled PPB (monocyclic) or BiPPB (bicyclic: 10 µg/ml) at room temperature. To block the binding, anti-PDGF-βR IgG was added to the cells 1h before FITC coupled PPB or BiPPB. After 2 h, cells were washed 3 to 4 times with cold PBS and fixed with 4% paraformaldehyde. The nuclei were counterstained with DAPI and mounted in citifluor (anti-fade reagent) and visualized under fluorescent microscope.

Similar binding experiments were performed in primary freshly isolated human myofibroblasts, mouse 3T3 fibroblasts and human hepatic stellate cells (LX2). For human Hepatic stellate cells, the sequence of BiPPB was as follows:
C(1)S R N L I D C(1) KGSGSGG C(2)S R N L I D C(2) :
   Cys(1)-Cys(1) and Cys(2)-Cys(2) disulfide bridge cyclisations

**Results:** The FITC-coupled monocyclic PPB did not show any binding as the PDGF-receptor requires dimeric interaction. FITC-coupled BiPPB showed significant binding to the cell type tested, which was almost completely blocked by PDGF receptor antibody, showing the receptor specificity of the binding to these cells (data not shown).

## Claims

1. An analog of interferon gamma (IFNγ), wherein the moiety mediating binding to its natural receptor is at least functionally disrupted and wherein the analog comprises a signaling moiety capable of mediating intracellular IFNγ activity, wherein the signaling moiety comprises at the C-terminal end of the analog a sequence selected from the group consisting of
(a) the amino acid sequence KFEVNNPQVQRQAFNELIRVVHQLLPESSLRKRKRSR;
(b) the amino acid sequence YSVTDLNVQRKAIHELIQVMAELSPAAKTGKRKRSQ;
(c) an amino acid sequence showing at least 90% identity to (a) or (b) provided that the intracellular signaling activity is maintained;
(d) the amino acid sequence under (a) or (b) wherein at most 5 amino acid residues are deleted, added or substituted, provided that the signaling activity, for example nuclear translocation, is maintained;
(e) the consensus sequence Vxxxx[V/I]Q[R/H][Q/K]A[F/V/I][N/H]ELI[R/Q] Vx[H/A][Q/E]L[L/S]P[E/A][S/A][S/A][L/K]xxKRKRS wherein x is any amino acid residue; and
(f) the sequence Xaa¹Xaa²Xaa³Xaa⁴ Val Xaa⁵ Xaa⁶ Xaa⁷ Xaa⁸ Xaa⁹ Gln Xaa¹⁰ Xaa¹¹ Ala Xaa¹² Xaa¹³ Glu Leu Ile Xaa¹⁴ Val Xaa¹⁵ Xaa¹⁶ Xaa¹⁷ Leu Xaa¹⁸ Pro Xaa¹⁹ Xaa²⁰ Xaa²¹ Xaa²² Xaa²³Lys Arg Lys Arg Ser Xaa²⁴ Xaa²⁵, wherein
Xaa¹, Xaa², Xaa³ Xaa⁴ Xaa⁶ Xaa¹¹, Xaa¹³, Xaa¹⁴, Xaa¹⁶ Xaa¹⁷, Xaa¹⁸ Xaa¹⁹ Xaa²⁰ Xaa²¹ Xaa²² Xaa²³ Xaa²⁴ and Xaa²⁵ is any amino acid residue Xaa⁵ is Asn or Thr
Xaa⁷ is Pro or Leu
Xaa⁸ is Gln or Asn
Xaa⁹ Val, Ile or Leu
Xaa¹⁰ is Arg, His or Lys
Xaa¹² is Phe, Val or Ile
Xaa¹⁵ is Val, Ile or Met
said signaling moiety being provided at its N-terminus, optionally via a linker, with at least one targeting domain capable of binding to a cell surface receptor other than the IFNγ receptor,
wherein said targeting domain can bind to the PDGFβ receptor, the mannose 6-phosphate/insulin-like growth factor-II receptor (M6P/ IGF2R), the asialoglycoprotein (ASGP) receptor or the mannose receptor (CD 206).

2. Analog according to claim 1, wherein the signaling moiety comprises the amino acid sequence KFEVNNPQVQRQAFNELIRVVHQLLPESSLRKRKRSR or YSVTDLNVQRKAIHELIQVMAELSPAAKTGKRKRSQ.

3. Analog according to claim 1 or 2, wherein the targeting domain is the cell adhesion peptide RGD.

4. Analog according to claim 1 or 2, wherein the targeting domain is an oligosaccharide, preferably mannose(-6-phosphate) or lactose, conjugated to a carrier molecule.

5. Analog according to any one of the preceding claims, wherein the targeting domain comprises two copies of the amino acid sequence X₁SRNLIDX₂, wherein X₁ and X₂ denote moieties which together can form a (peptidic) bond such that a cyclic structure is formed wherein the sequence SRNLID is part of the ring and wherein the two copies are spaced by a linker sequence of 2 to 7 amino acids.

6. Analog according to claim 5 wherein the targeting domain comprises the amino acid sequence CSRNLIDC-linker- CSRNLIDCS, wherein the linker is an amino acid sequence of 3 to 7, preferably 4 or 5, amino acid residues.

7. Analog according to claim 5 or 6, wherein the linker is selected from the group consisting of (i) the sequences K(GS)ₘGG wherein m is 1 or 2 and (ii) the sequences of the general formula [G ₙ D ₘ] wherein n+m is from 4 to 7, wherein n ≥ 4 and M an integer between 0 and 3.

8. Analog according to claim 7, wherein the targeting domain consists of or comprises the sequence CSRNLIDCGGGDGGCSRNLIDC, CSRNLIDCGGDGGCSRNLIDC, CSRNLIDCGDDGGCSRNLIDC or CSRNLIDCGGGGGGCSRNLIDC.

9. A conjugate comprising a compound of interest conjugated to a targeting domain, said targeting domain comprising the amino acid sequence X₁SRNLIDX₂-linker- X₃SRNLIDX₄, wherein the pair of X₁ and X₂ and the pair of X₃ and X₄ can form a bond, preferably a peptidic bond, such that a bicyclic structure is formed wherein the sequences SRNLID are each part of a ring, and wherein the linker is an amino acid sequence of 2 to 7, preferably 3 to 5, amino acid residues.

10. Analog or conjugate according to any one of the preceding claims, provided with at least one non-antigenic polymer, preferably selected from the group consisting of polyethylene glycols and derivatives thereof.

11. An isolated nucleic acid sequence encoding a proteinaceous analog or conjugate according to any one of the preceding claims.

12. An expression vector comprising an isolated nucleic acid sequence according to claim 11.

13. A host cell comprising a nucleic acid sequence according to claim 11 or a vector according to claim 12, preferably wherein said host cell is a bacterial or mammalian host cell.

14. A pharmaceutical composition comprising an analog or conjugate according to any one of claims 1 to 10 and a pharmaceutically acceptable carrier, adjuvant and/or excipient.

15. Use of an analog or conjugate according to any one of claims 1 to 10 for the manufacture of a medicament for the therapeutic or prophylactic treatment of a disease selected from cancer, fibrotic disease and sclerotic disease.

16. Analog or conjugate according to any one of claims 1 to 10, for use in the therapeutic or prophylactic treatment of a disease selected from cancer, fibrotic disease and sclerotic disease.

17. Analog or conjugate according to claim 16, for use in the treatment of a liver disease, preferably a chronic liver disease such as liver cirrhosis.

## Patentansprüche

1. Analogon von Interferon Gamma (IFNγ), wobei der Rest, der die Bindung an seinen natürlichen Rezeptor vermittelt, mindestens funktionell zerstört ist und wobei das Analogon einen Signalrest umfasst, der in der Lage ist, intrazelluläre IFNγ-Aktivität zu vermitteln, wobei der Signalrest am C-terminalen Ende des Analogons eine Sequenz umfasst, ausgewählt aus der Gruppe bestehend aus
(a) der Aminosäuresequenz KFEVNNPQVQRQAFNELIRVVHQLLPESSLRKRKRSR;
(b) der Aminosäuresequenz YSVTDLNVQRKAIHELIQVMAELSPAAKTGKRKRSQ;
(c) einer Aminosäuresequenz, die mindestens 90 % Identität zu (a) oder (b) aufweist, vorausgesetzt die intrazelluläre Signalaktivität wird aufrechterhalten;
(d) der Aminosäuresequenz unter (a) oder (b), wobei höchstens 5 Aminosäurereste entfernt, hinzugefügt oder substituiert werden, vorausgesetzt die Signalaktivität, zum Beispiel nukleare Translokation, wird aufrechterhalten;
(e) der Konsensussequenz
Vxxxx[V/I]Q[R/H] [Q/K]A[F/V/I] [N/H]ELI[R/Q]Vx[H/A] [Q/E]L[L/S]P[E/A] [S/A][S/A][L/K]xxKRKRS, wobei x ein beliebiger Aminosäurerest ist; und
(f) der Sequenz Xaa¹ Xaa² Xaa³ Xaa⁴ Val Xaa⁵ Xaa⁶ Xaa⁷ Xaa⁸ Xaa⁹ Gln Xaa¹⁰Xaa¹¹ Ala Xaa¹²Xaa¹³ Glu Leu Ile Xaa¹⁴ Val Xaa¹⁵ Xaa¹⁶ Xaa¹⁷ Leu Xaa¹⁸ Pro Xaa¹⁹ Xaa²⁰ Xaa²¹ Xaa²² Xaa²³ Lys Arg Lys Arg Ser Xaa²⁴ Xaa²⁵,
wobei
Xaa¹, Xaa², Xaa³, Xaa⁴, Xaa⁶, Xaa¹¹, Xaa¹³, Xaa¹⁴, Xaa¹⁶, Xaa¹⁷, Xaa¹⁸, Xaa¹⁹, Xaa²⁰, Xaa²¹, Xaa²², Xaa²³, Xaa²⁴ und Xaa²⁵ irgendein Aminosäurerest ist
Xaa⁵ Asn oder Thr ist
Xaa⁷ Pro oder Leu ist
Xaa⁸ Gln oder Asn ist
Xaa⁹ Val, Ile oder Leu ist
Xaa¹⁰ Arg, His oder Lys ist
Xaa¹² Phe, Val oder Ile ist
Xaa¹⁵ Val, Ile oder Met ist
der Signalrest an seinem N-Terminus bereitgestellt wird, optional über einen Linker, mit mindestens einer Zieldomäne, die in der Lage ist, an einen anderen Zelloberflächenrezeptor als den IFNγ-Rezeptor zu binden,
wobei die Zieldomäne an den PDGFβ-Rezeptor, den Mannose 6-phosphat/insulinähnlichen Wachstumsfaktor-II-Rezeptor (M6P/ IGF2R), den Asialoglycoprotein (ASGP) Rezeptor oder den Mannose-Rezeptor (CD206) binden kann.

2. Analogon nach Anspruch 1, wobei der Signalrest die Aminosäuresequenz KFEVNNPQVQRQAFNELIRVVHQLLPESSLRKRKRSR oder YSVTDLNVQRKAIHELIQVMAELSPAAKTGKRKRSQ umfasst.

3. Analogon nach Anspruch 1 oder 2, wobei die Zieldomäne das Zelladhäsionspeptid RGD ist.

4. Analogon nach Anspruch 1 oder 2, wobei die Zieldomäne ein Oligosaccharid, bevorzugt Mannose-6-phosphat oder Lactose, konjugiert an ein Trägermolekül, ist.

5. Analogon nach einem der vorhergehenden Ansprüche, wobei die Zieldomäne zwei Kopien der Aminosäuresequenz X₁SRNLIDX₂ umfasst, wobei X₁ und X₂ Reste bezeichnen, die zusammen eine (peptidische) Bindung bilden können, sodass eine zyklische Struktur gebildet wird, wobei die Sequenz SRNLID Teil des Rings ist und wobei die zwei Kopien durch eine Linkersequenz von 2 bis 7 Aminosäuren voneinander beabstandet sind.

6. Analogon nach Anspruch 5, wobei die Zieldomäne die Aminosäuresequenz CSRNLIDC-Linker- CSRNLIDCS umfasst, wobei der Linker eine Aminosäuresequenz von 3 bis 7, bevorzugt 4 oder 5, Aminosäureresten ist.

7. Analogon nach Anspruch 5 oder 6, wobei der Linker ausgewählt ist aus der Gruppe bestehend aus (i) den Sequenzen K(GS)ₘGG, wobei m 1 oder 2 ist, und (ii) den Sequenzen der allgemeinen Formel [GₙDₘ], wobei n+m von 4 bis 7 ist, wobei n ≥ 4 und M eine ganze Zahl zwischen 0 und 3 ist.

8. Analogon nach Anspruch 7, wobei die Zieldomäne aus der Sequenz CSRNLIDCGGGDGGCSRNLIDC, CSRNLIDCGGDGGCSRNLIDC, CSRNLIDCGDDGGCSRNLIDC oder CSRNLIDCGGGGGGCSRNLIDC besteht oder diese umfasst.

9. Konjugat, umfassend eine Verbindung von Interesse, konjugiert an eine Zieldomäne, welche Zieldomäne die Aminosäuresequenz X₁SRNLIDX₂-Linker- X₃SRNLIDX₄ umfasst, wobei das Paar X₁ und X₂ und das Paar X₃ und X₄ eine Bindung bilden können, bevorzugt eine peptidische Bindung, sodass eine bizyklische Struktur gebildet wird, wobei die Sequenzen SRNLID jeweils Teil eines Rings sind und wobei der Linker eine Aminosäuresequenz von 2 bis 7, bevorzugt 3 bis 5, Aminosäureresten ist.

10. Analogon oder Konjugat nach einem der vorhergehenden Ansprüche, bereitgestellt mit mindestens einem nicht-antigenen Polymer, bevorzugt ausgewählt aus der Gruppe bestehend aus Polyethylenglykolen und Derivaten davon.

11. Isolierte Nukleinsäuresequenz, kodierend ein proteinartiges Analogon oder Konjugat nach einem der vorhergehenden Ansprüche.

12. Expressionsvektor, umfassend eine isolierte Nukleinsäuresequenz nach Anspruch 11.

13. Wirtszelle, umfassend eine Nukleinsäuresequenz nach Anspruch 11 oder einen Vektor nach Anspruch 12, bevorzugt wobei die Wirtszelle eine Bakterien- oder Säuger-Wirtszelle ist.

14. Pharmazeutische Zusammensetzung, umfassend ein Analogon oder Konjugat nach einem der Ansprüche 1 bis 10 und einen pharmazeutisch verträglichen Träger, Hilfsstoff und/oder Exzipienten.

15. Verwendung eines Analogons oder Konjugats nach einem der Ansprüche 1 bis 10 zur Herstellung eines Arzneimittels für die therapeutische oder prophylaktische Behandlung einer Erkrankung, ausgewählt aus Krebs, fibrotischer Erkrankung und sklerotischer Erkrankung.

16. Analogon oder Konjugat nach einem der Ansprüche 1 bis 10 zur Verwendung in der therapeutischen oder prophylaktischen Behandlung einer Erkrankung, ausgewählt aus Krebs, fibrotischer Erkrankung und sklerotischer Erkrankung.

17. Analogon oder Konjugat nach Anspruch 16 zur Verwendung bei der Behandlung einer Lebererkrankung, bevorzugt einer chronischen Lebererkrankung wie etwa Leberzirrhose.

## Revendications

1. Un analogue de l'interféron gamma (IFNγ), dans lequel le fragment servant de médiateur à la liaison à son récepteur naturel est au moins fonctionnellement perturbé, et dans lequel l'analogue comprend un fragment de signalisation capable de médier l'activité d'IFNγ intracellulaire, ledit fragment de signalisation comprenant à son extrémité C-terminale de l'analogue, une séquence choisie dans le groupe consistant en
(a) la séquence d'acides aminés **KFEVNNPQVQRQAFNELIRWHQLLPESSLRKRKRSR;**
(b) la séquence d'acides aminés **YSVTDLNVQRKAIHELIQVMAELSPAAKTGKRKRSQ;**
(c) une séquence d'acides aminés présentant au moins 90% d'identité avec a) ou b) à condition que l'activité de signalisation intracellulaire soit maintenue ;
(d) la séquence d'acides aminés a) ou b) précitée dans laquelle au moins 5 résidus d'acides aminés sont supprimés, ajoutés ou substitués, à condition que l'activité de signalisation, par exemple une translocation nucléaire soit maintenue.
(e) la séquence consensus 
VXXXX[V/I]Q[R/H][Q/K]A[FN/I][N/H]ELI[R/Q]VX[H/A][Q/E]L[L/S]P[E/A][S/A][S/A][L/K]X
XKRKRS
 dans laquelle x est un résidu d'acides aminés quelconque, et 1 2 3 4 56789 9
(f) la séquence Xaa¹Xaa² Xaa³Xaa Val Xaa⁵ Xaa⁶ Xaa⁷ Xaa⁸ Xaa⁹ Gln Xaa¹⁰ Xaa¹¹ Ala Xaa¹² Xaa¹³ Glu Leu Ile Xaa¹⁴ Val Xaa¹⁵ Xaa¹⁶ Xaa¹⁷ 18 19 20 21 22 23 24 Leu Xaa¹⁸ Pro Xaa¹⁹ Xaa²⁰ Xaa²¹ Xaa²² Xaa²³ Lys Arg Lys Arg Ser Xaa²⁴ 25 Xaa²⁵, dans laquelle :
Xaa¹, Xaa², Xaa³ Xaa⁴ Xaa⁶ Xaa¹¹, Xaa¹³, Xaa¹⁴, Xaa¹⁶ Xaa¹⁷, Xaa¹⁸ Xaa¹⁹ Xaa²⁰ Xaa²¹ Xaa²²Xaa²³ Xaa²⁴ et Xaa²⁵ est n'importe quel résidu d'acides aminés 5
Xaa⁵ est Asn ou Thr,
Xaa⁷ est Pro ou Leu
Xaa⁸ est Gln ou Asn
Xaa est Val, Ile ou Leu
Xaa¹⁰ est Arg, His ou Lys 12
Xaa est Phe, Val ou Ile 15
Xaa est Val, Ile ou Met
ledit fragment de signalisation étant pourvu à son extrémité N-terminale, éventuellement par l'intermédiaire d'un coupleur, d'au moins un domaine de ciblage capable de se lier à un récepteur de surface cellulaire autre que le récepteur de l'IFNγ
dans lequel ledit domaine de ciblage se lie au récepteur PDGFB, au récepteur mannose 6-phosphate/facteur de croissance II de type insuline (M6P/IGF2R), au récepteur asialoglycoprotéine (ASGP) ou au récepteur mannose (CD 206).

2. Analogue selon la revendication 1, dans lequel le fragment de signalisation comprend la séquence d'acides aminés **KFEVNNPQVQRQAFNELIRVVHQLLPESSLRKRKRSR** ou **YSVTDLNVQRKAIHELIQVMAELSPAAKTGKRKRSQ.**

3. Analogue selon la revendication 1 or 2, dans lequel le domaine de ciblage est le peptide d'adhésion cellulaire RGD.

4. Analogue selon la revendication 1 or 2, dans lequel le domaine de ciblage est un oligosaccharide, de préférence mannose (-6-phosphate) ou lactose, conjugué à une molécule de support.

5. Analogue selon une quelconque des revendications précédentes, dans lequel le domaine de ciblage comprend deux copies de la séquence d'acides aminés X₁SRNLIDX₂, où X₁ et X₂ correspondent à des fragments qui ensemble peuvent former une liaison (peptidique) telle qu'une structure cyclique soit formée, la séquence SRNLID étant partie du cycle et les deux copies étant séparées par une séquence de coupleur formée de 2 à 7 acides aminés.

6. Analogue selon la revendication 5 dans lequel le domaine de ciblage comprend la séquence d'acides aminés CSRNLIDC-coupleur-CSRNLIDCS, où le coupleur est une séquence d'acides aminés formée de 3 à 7, de préférence 4 ou 5, résidus d'acide aminé.

7. Analogue selon la revendication 5 ou 6, dans lequel le coupleur est choisi dans le groupe consistant en
(i) les séquences K(GS)ₘGG dans lequel m est lor 2, et
(ii) les séquences de formule générale [GₙDₘ] dans lequel n+m est compris entre 4 et 7, dans lequel n est ≥4 et M est un nombre entier compris entre 0 et 3.

8. Analogue selon la revendication 7, dans lequel le domaine de ciblage consiste en ou comprend la séquence CSRNLIDCGGGDGGCSRNLIDC, CSRNLIDCGGDGGCSRNLIDC, CSRNLIDCGDDGGCSRNLIDC ou CSRNLIDCGGGGGGCSRNLIDC.

9. Un conjugué comprenant un composé d'intérêt conjugué à un domaine de ciblage, ledit domaine de ciblage comprenant la séquence d'acides aminés X₁SRNLIDX₂-coupleur- X₃SRNLIDX₄, ou la paire X₁ et X₂ et la paire of X₃ et X₄ peuvent former une liaison, de préférence une liaison peptidique, telle qu'une structure bicyclique se forme où les séquences SRNLID font chacune partie d'un cycle, et dans lequel le coupleur est une séquence d'acides aminés comprenant de 2 à 7, préférablement 3 à 5, résidus d'acide aminé.

10. Analogue ou conjugué selon une quelconque des revendications précédentes, pourvu d'au moins un polymère non-antigénique, préférablement choisi dans le groupe consistant en polyéthylène glycols et leurs dérivés.

11. Une séquence d'acide nucléique isolée codant pour un analogue ou conjugué protéique selon une quelconque des revendications précédentes.

12. Un vecteur d'expression comprenant une séquence d'acide nucléique isolée selon la revendication 11.

13. Une cellule hôte comprenant une séquence d'acide nucléique selon la revendication 11 ou un vecteur selon la revendication 12, de préférence dans lequel ladite cellule hôte est une cellule hôte d'une bactérie ou d'un mammifère.

14. Une composition pharmaceutique comprenant un analogue ou conjugué selon une quelconque des revendications 1 à 10 et un support, adjuvant et/ou excipient pharmaceutiquement acceptable.

15. Utilisation d'un analogue ou conjugué selon une quelconque des revendications 1 à 10 pour la fabrication d'un médicament pour le traitement thérapeutique ou prophylactique d'une affection du groupe du cancer, des affections fibrotiques et des affections sclérotiques.

16. Analogue ou conjugué selon une quelconque des revendications 1 à 10, pour une utilisation pour le traitement thérapeutique ou prophylactique d'une affection du groupe du cancer, des affections fibrotiques et des affections sclérotiques.

17. Analogue ou conjugué selon la revendication 16, pour une utilisation pour le traitement d'une maladie du foie, de préférence une maladie chronique du foie, telle la cirrhose du foie.
